# EUROPEAN PATENT SPECIFICATION

(11) **EP 1 276 483 B1**
(45) Date of publication and mention of the grant of the patent: **02.09.2009**
(21) Application number: 00922395.9
(22) Date of filing: 28.04.2000
(51) Int. Cl.: A61K 31/662, A61K 31/10, A61K 31/33, A61P 25/28

(54) **COMPOSITIONS AND METHODS FOR TREATING AMYLOIDOSIS USING SULPHONATE DERIVATIVES**
ZUSAMMENSETZUNGEN UND VERFAHREN ZUR BEHANDLUNG VON AMYLOIDOSIS MIT SULPHONATDERIVATEN
COMPOSITIONS ET PROCEDES POUR TRAITER L'AMYLOSE PAR DES SULPHONATES

(30) Priority: 28.04.1999 US 131464 P; 24.05.1999 US 135545 P; 09.07.1999 US 143123 P
(43) Date of publication of application: 22.01.2003
(73) Proprietor: BELLUS Health (International) Limited, 1015 Lausanne (CH)
(72) Inventor: GORDON, Heather, St. Catharines, Ontario L2S 1Z3 (CA); SZAREK, Walter, Kingston, Ontario K7L 2Y6 (CA); WEAVER, Donald, Halifax, Nova Scotia B3M 3R4 (CA); KONG, Xianqi, Dollard-des-Ormeaux H9B 3J7 (CA)
(74) Representative: Bachelin, Karl Martin Raimund
(86) International application number: PCT/CA2000/000494
(87) International publication number: WO 2000/064420

(56) References cited:
- WO-A-99/06545
- WO-A-99/08685
- WO-A-99/40909
- US-A- 5 643 562
- POLLACK S J ET AL: "SULFONATED DYES ATTENUATE THE TOXIC EFFECTS OF BETA-AMPLOID IN A STRUCTURE-SPECIFIC FASHOIN" NEUROSCIENCE LETTERS,IE,LIMERICK, vol. 197, 1995, pages 211-214, XP000612230 ISSN: 0304-3940

## Description

This application claims the benefit of priority under 35 U.S.C. 119(e) to copending U.S. Provisional Application No. 60/131,464, filed on April 28, 1999, U.S. Provisional Application No. 60/135,545, filed on May 24, 1999, and U.S. Provisional Application No. 60/143,123, filed on July 9, 1999. This application is also related to U.S. Patent No. 5972328, issued October 26, 1999.

### BACKGROUND OF THE INVENTION

Amyloidosis refers to a pathological condition characterized by the presence of amyloid. "Amyloid" is a generic term referring to a group of diverse but specific extracellular protein deposits which are seen in a number of different diseases. Though diverse in their occurrence, all amyloid aggregates have common morphologic properties, stain with specific dyes (e.g., Congo red), and have a characteristic red-green birefringent appearance in polarized light after staining. They also share common ultrastructural features and common x-ray diffraction and infrared spectra.

Amyloidosis can be classified clinically as primary, secondary, familial and/or isolated. Primary amyloidosis appears *de novo* without any preceding disorder. Secondary amyloidosis is that form which appears as a complication of a previously existing disorder. Familial amyloidosis is a genetically inherited form found in particular geographic populations. Isolated forms of amyloidosis are those that tend to involve a single organ system. Different amyloids are also characterized by the type of protein present in the aggregate. For example, neurodegenerative diseases such as scrapie, bovine spongiform encephalitis, Creutzfeldt-Jakob disease, transmissible spongiform encephalitis ("TSE"), and the like are characterized by the appearance and accumulation of a protease-resistant form of a prion protein (referred to as AScr or PrP-27) in the central nervous system.

Similarly, Alzheimer's disease, another neurodegenerative disorder, is characterized by congophilic angiopathy, neuritic plaques and neurofibrillary tangles, all of which have the characteristics of amyloids. In this case, the plaques and blood vessel amyloid are formed by the beta protein. Other systemic diseases such as adult-onset diabetes, complications of long-term hemodialysis and sequelae of long-standing inflammation or plasma cell dyscrasias are characterized by the accumulation of amyloids systemically. In each of these cases, a different amyloidogenic protein is involved.

Other harmful effect of amyloidosis include toxicity to cells by the presence of greater than normal levels of amyloid *in vivo.* It has been noted that once amyloid fibrils are assembled into fibers, e.g., amyloid aggregation, the fibers are known to be toxic to nerve cells and present a risk to the viability of those cells. So in addition to the noted detrimental effects of amyloid plaques *in vivo*, the presence of amyloid itself can be harmful to the organism.

We are aware of United States patent US 5,643,562 (KISILEVSKY et al.) which describes therapeutic compounds and methods for inhibiting amyloid deposition in a subject, whatever its clinical setting. Amyloid deposition is inhibited by the administration to a subject of an effective amount of a therapeutic compound comprising an anionic group and a carrier molecule, or a pharmaceutically acceptable salt thereof, such that an interaction between an amyloidogenic protein and a basement membrane constituent is inhibited. Preferred anionic groups are sulfonates and sulfates. Preferred carrier molecules include carbohydrates, polymers, peptides, peptide derivatives, aliphatic groups, alicyclic groups, heterocyclic groups, aromatic groups and combinations thereof.

We are also aware of Pollack S J et al: "Sulfonated dyes attenuate the toxic effects of beta-amploid in a structure-specific fashoin" Neuroscience Letters,IE,Limerick, vol. 197, 1995, pages 211-214, which discloses that some sulphate containing glycosaminoglycans attenuate the toxic effects of some β-amyloid fragments. The protective effect appears specific for compounds whose sulfonate groups can interact with the β-pleated structure of aggregated amyloid. It is suggested that by binding β-amyloid these compounds may prevent toxic interactions of the peptide with cells.

### SUMMARY OF THE INVENTION

Essential and optional features of the present invention are set out in the accompanying main- and sub-claims respectively.

This the present invention provides compositions for use in the treatment of amyloidosis. In particular, compositions are disclosed for inhibiting, preventing and treating amyloid aggregation. e.g., in pancreatic islets wherein the amyloidotic aggregates to be treated are, in an embodiment, islet amyloid polypeptide (IAPP)- associated amyloid aggregates, e.g., having at least some β-sheet structure. Accordingly, the compositions of the invention are used for inhibiting amyloidosis in disorders in which such amyloid aggregation occurs.

A method described herein involves administering *in vivo* or *ex vivo* an effective amount of a therapeutic compound having the formula (i): 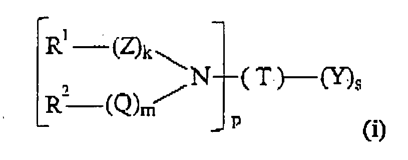 or a pharmaceutically acceptable salt or ester, such that modulation of amyloid aggregation occurs.
R¹ is
   a straight or branched C₁₋₂₂ alkyl group, a straight or branched C₁₋₂₂ alkenyl group, a straight or branched C₁₋₂₂ alkynyl group, each of which may be substituted on one or more carbons with a halogen, a hydroxyl, a thiol, an amino, an alkoxy, an alkylcarboxy, an alkylthio or a nitro group, or
   an alicyclic group selected from cyclopropane, cyclopentane, cycloolefins and fused ring structures, each of which alicyclic groups may be substituted with one or more substituents selected from C₁₋₆ alkyl, C₁₋₆ alkoxy, C₁₋₆ alkylthio, C₁₋₆ alkylamino, C₁₋₆ alkylcarboxy, nitro, hydroxy. -CF₃, and -CN. or
   a heterocyclic group, which is saturated or unsaturated, can include fused ring structures and can be substituted at one or more constituent atoms with a halogen, a C₁₋₆ alkyl, a C₁₋₆ alkenyl, a C₁₋₆ alkoxy, a C₁₋₆ alkylthio, a C₁₋₆ alkylamino, a C₁₋₆ alkylcarboxyl, a nitro, a hydroxyl, -CF₃, or -CN, or
   an aryl group selected from 5-membered single-ring groups which may include from zero to four heteroatoms and 6-membered single-ring groups selected from benzene, pyrazine, pyridazine and pyrimidine, wherein said aryl group may be substituted at one or more ring positions with substituents elected from halogen, C₁₋₆, alkyl, C₁₋₆ alkenyl, C₁₋₆ alkoxy, C₁₋₆ alkylthio, C₁₋₆ alkylamino, C₁₋₆ alkylcarboxyl, nitro, hydroxyl, -CF₃, and -CN;
R² is a hydrogen atom or a substituted or unsubstituted aliphatic or aryl group;
Z and Q are each independently a carbonyl (C=O), thiocarbonyl (C=S), sulfonyl (SO₂), or sulfoxide (S=O);
k and m arc 0 or 1, provided when m is 1, R² is not a hydrogen atom;
p and s are each independently 1 to 2;
T is a group of the formula -(CD¹D²)ₙ-, wherein n is an integer from 1 to 25, C is carbon and D¹ and D² are independently hydrogen or halogen atoms; aliphatic, aromatic or heterocyclic groups; alkylamino or arylamino; or alkyloxy or aryloxy;
or R¹ and R² are taken together w form an alkylene group, k and m arc zero, p and s are each one, and T is an alkylene group; and
Y is -A-X, wherein A is SO₃ and X is hydrogen or a cation. In a preferred embodiment, the therapeutic compounds disclosed herein prevent or inhibit amyloid aggregation.

The methods described herein involve, in an embodiment, administering to a subject a therapeutic compound which inhibits, reduces or disrupts amyloid deposits, e.g., LAPF-associated amyloids deposits.

In a preferred embodiment, therapeutic compounds in accordance with the present disclosure include those where, within the scope of the definitions of appended claim 1, R¹ is an alkyl, alkenyl, or aryl group, k is one, Z is a carbonyl group, R² is a hydrogen atom or an alkyl group, m is zero, p and s are 1, T is an alkylene group, and Y is SO₃X wherein X is H⁺ or other cation such as cations of alkali metals. In another embodiment a group of therapeutic compounds include those where, within the scope of the definitions of appended claim 1, R¹ and R² are alkyle, alkenyl, or aryl groups, or R¹ and R² are taken together to form an alkylene group, k and m are each one, Z and Q are carbonyl groups, p and s are 1, T is an alkylene group, and Y is SO₃X where X is H⁺ or other cation such as cations of alkali metals.

In an further preferred embodiment, therapeutic compounds in accordance with the present disclosure include those where, within the scope of the definitions of appended claim 1, R¹ is an alkyl, alkenyl, or aryl group, k and m are zero, R² is hydrogen or an alkyl group, p and s are each one, T is an alkylene group, and Y is SO₃X, wherein X is H⁺ or another cation, such as alkali metal cations. In another embodiment, therapeutic compounds include those where R¹ and R² are alkyl, alkenyl, or aryl groups, or R¹ and R² are taken together to form an alkylene group, k and m are zero, p and s are each one, T is an alkylene group, Y is SO₃X, where X is H⁺ or another cation, such as alkali metal cations.

The therapeutic compounds disclosed herein are administered to a subject by a route which is effective for modulation of amyloid aggregation. Suitable routes of administration include subcutaneous, intravenous and intraperitoneal injection. The therapeutic compounds of the invention have been found to be effective when administered orally. Accordingly, a preferred route of administration is oral administration. The therapeutic compounds can be administered with a pharmaceutically acceptable vehicle.

Methods are also disclosed herein for treating a disease state associated with amyloidosis by administering to a subject an effective amount of a therapeutic compound having the formula described *supra*, such that a disease state associated with amyloidosis is treated.

The invention further provides pharmaceutical compositions for treating amyloidosis. The pharmaceutical compositions include a therapeutic compound of the invention in an amount effective to modulate amyloid aggregation and a pharmaceutically acceptable vehicle.

Packaged pharmaceutical compositions for treating amyloidosis include a therapeutic compound of the invention and instructions for using the pharmaceutical composition for treatment of amyloidosis.

### BRIEF DESCRIPTION OF THE DRAWING

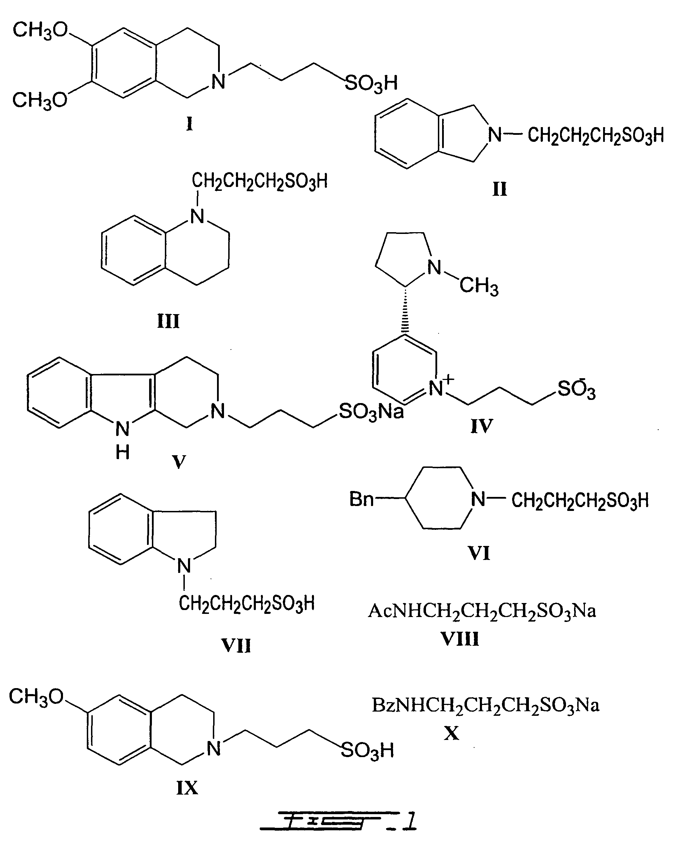

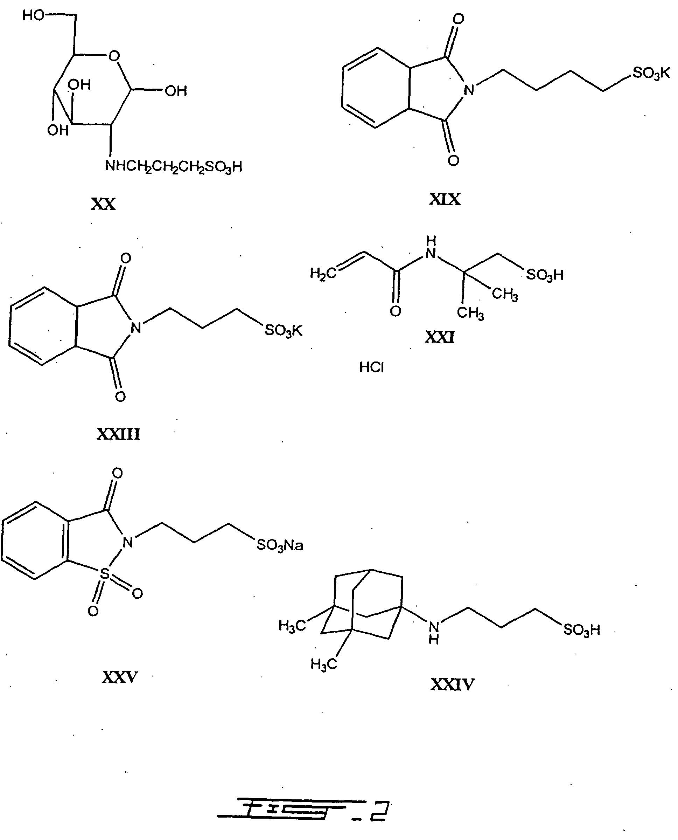

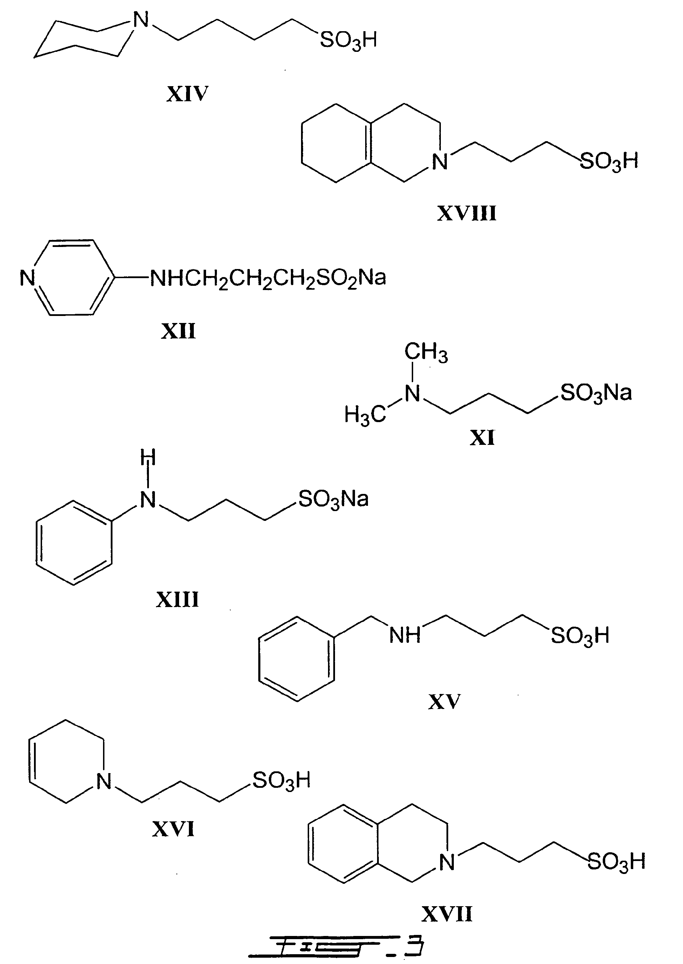

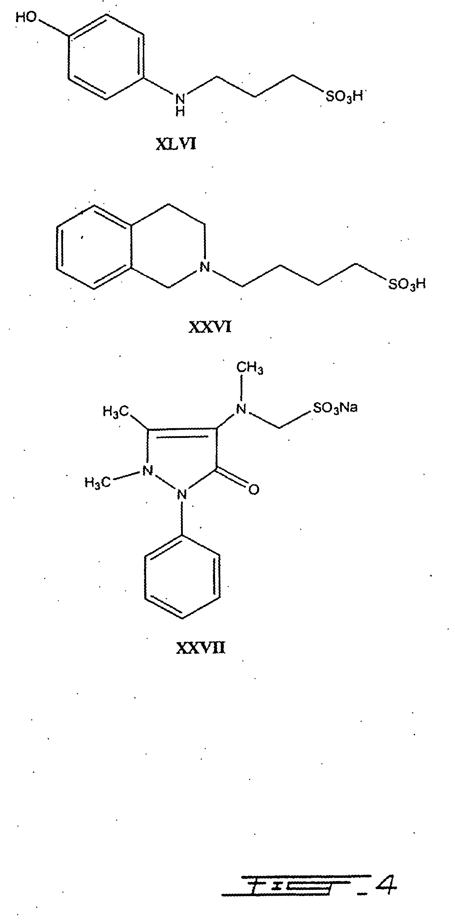

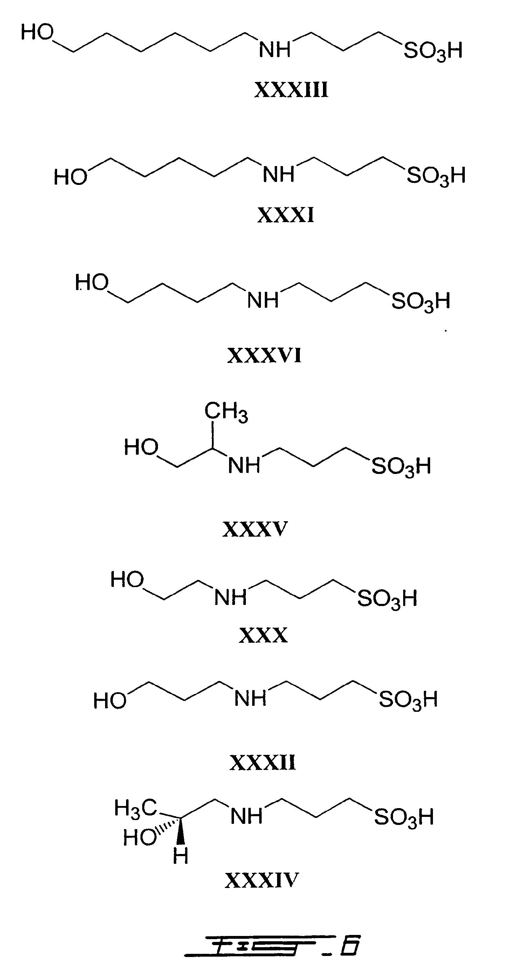

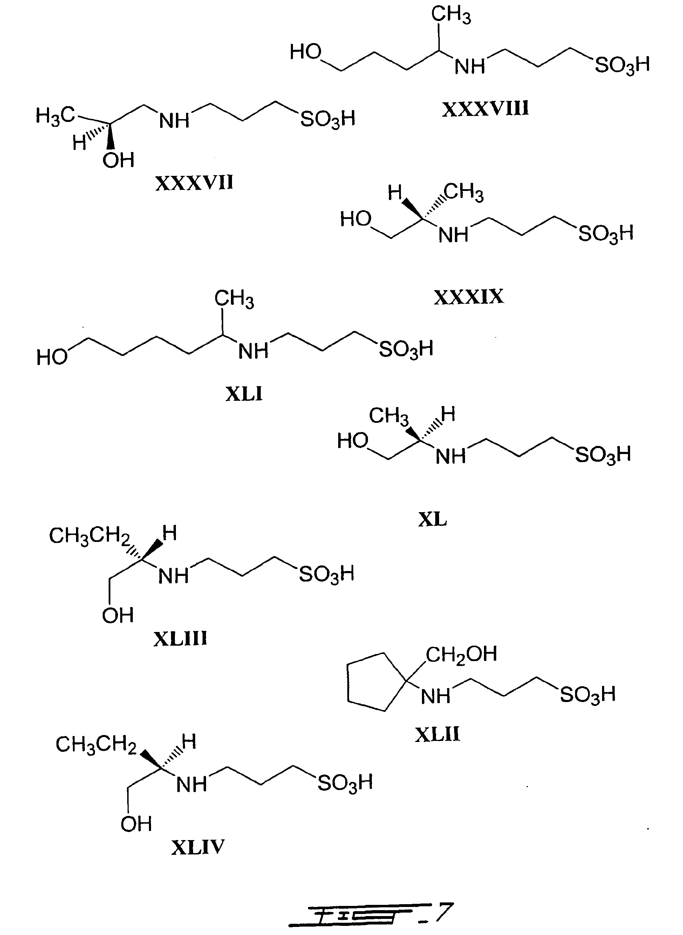

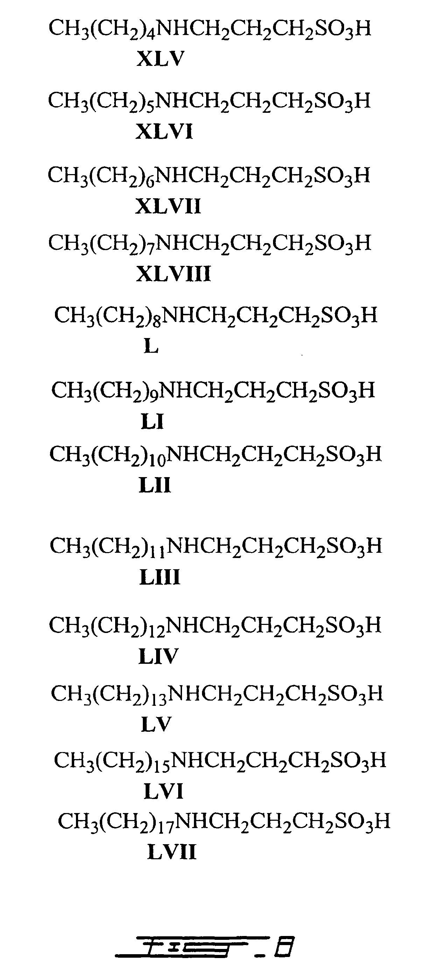

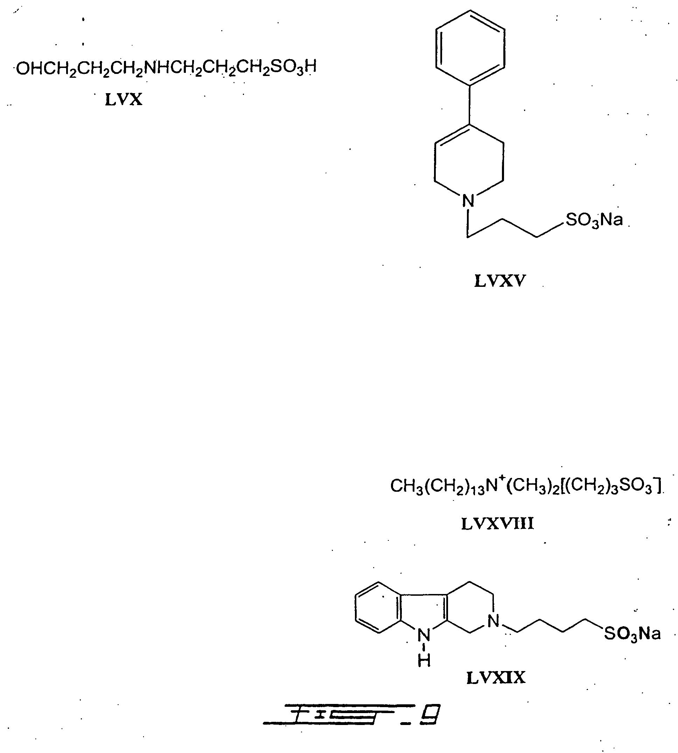

Figures 1-9 depict exemplary chemical structures of compounds described in the specification.
Figure 10 is the ¹H NMR spectrum of 8-methoxy-5-quinolinesulfonic acid, sodium salt (in DMSO-*d₆*), made as in Example 9.
Figures 11 and 12 are histograms showing the effectiveness of compounds of the invention, **XXVII** and **XVII,** respectively in an acute animal model for secondary amyloidosis, in accordance with Example 5.

### DETAILED DESCRIPTION OF THE INVENTION

The present disclosure pertains to methods and compositions useful for treating amyloidosis. The disclosed methods involve administering to a subject a therapeutic compound which modulates amyloid aggregation. "Modulation of amyloid aggregation" is intended to encompass prevention or stopping of amyloid formation, inhibition or slowing down of further amyloid aggregation in a subject with ongoing amyloidosis, e.g., already having amyloid aggregates, and reducing or reversing of amyloid aggregates in a subject with ongoing amyloidosis. Modulation of amyloid aggregation is determined relative to an untreated subject or relative to the treated subject prior to treatment. "Amyloid" includes IAPP-associated amyloid, including, but not limited to, β-sheet amyloid assembled substantially from IAPP subunits as well as other types f amyloid-related diseases such as Alzheimer's Disease and systemic amyloid disorders.

In one embodiment, a method described herein includes administering to the subject an effective amount of a therapeutic compound which has at least one anionic group covalently attached to a linking group. The therapeutic compound has the formula **(i):** 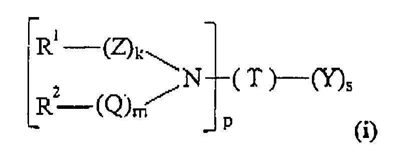 R¹ is or a pharmaceutically acceptable salt or ester thereof.
R¹ is a straight or branched C₁₋₂₂ alkyl group, a straight or branched C₁₋₂₂ alkenyl group, a straight or branched C₁₋₂₂ alkynyl group, each or which may be substituted on one or more carbons with a halogen, a hydroxyl, a thiol, an amino, an alkoxy, an alkylcarboxy, an alkylthio or a nitro group, or an alicyclic group selected from cyclopropane, cyclopentane, cycloolefins and fused ring structures, each of which alicyclic groups may be substituted with one or more substituents selected from C₁₋₆ alkyl, C₁₋₆ alkoxy, C₁₋₆ alkylthio, C₁₋₆ alkylamino, C₁₋₆ alkylcarboxyl, nitro, hydroxyl, -CF₃, and -CN, or
   a heterocyclic group, which is saturated or unsaturated, can include fused ring structures and can be substituted at one or more constituent atoms with a halogen, a C₁₋₆ alkyl, a C₁₋₆ alkenyl, a C₁₋₆ alkoxy, a C₁₋₆ alkylthio, a C₁₋₆ alkylamino, a C₁₋₆ alkylcarboxyl, a nitro, a hydroxyl, -CF₃, or -CN, or
   an aryl group selected from 5-membered single-ring groups which may include from zero to four heteroatoms and 6-membered single-ring groups selected from benzene, pyrazine, pyridazine and pyrimidine, wherein said aryl group may be substituted at one or more ring positions with substituents selected from halogen, C₁₋₆ alkyl, C₁₋₆ alkenyl, C₁₋₆ alkoxy, C₁₋₆ alkylthio. C₁₋₆ alkylamino, C₁₋₆ alkylcarboxyl, nitro, hydroxy, -CF₃, and -CN;
R² is a hydrogen atom or a substituted or unsubstituted aliphatic or aryl group;
Z and Q arc each independently a carbonyl (C=O), thiocarbonyl (C=S), sulfonyl (SO₂), or sulfoxide (S=O);
k and m arc 0 or 1, provided when m is 1, R² is not a hydrogen atom,
p and s are each independently 1 to 2;
T is a group of the formula -(CD¹D²)ₙ-, wherein n is an integer from 1 to 25, C is carbon and D¹ and D² arc independently hydrogen or halogen atoms; aliphatic, aromatic or heterocyclic groups: alkylamino or arylamino; or alkyloxy or aryloxy;
or R¹ and R² arc taken together to form an alkylene group, k and m are zero, p and s arc each one, and T is an alkylene group: and
Y is -A-X, wherein A is SO₃ and X is hydrogen or a cation. In a preferred embodiment, the therapeutic compounds disclosed herein prevent or inhibit amyloid protein assembly into insoluble fibrils which, *in vivo*, are deposited in various organs. It is also believed, without limitation, that the compounds also prevent the amyloid protein, whether in soluble or non-soluble form, from binding or adhering to a cell surface and causing cell damage or toxicity.

The number of amino or amido groups and anionic groups (i.e., determined by "p" and "s") are each independently 1 to 2 selected such that the biodistribution of the compound for an intended target site is not prevented while maintaining activity of the compound. Further, p and s are selected such that a sufficient number of groups, Z, Q, T and/or Y, are presented for treatment of a disease or condition. For example, the number of anionic groups is not so great as to inhibit traversal of an anatomical barrier, such as a cell membrane, or entry across a physiological barrier, such as the blood-brain barrier, in situations where such properties are desired. The integers for p and s are 1 to 2. Linking group T is of the formula -(CD¹D²)ₙ-, wherein n is an integer from 1 to 25, C is carbon and D¹ and D² are independently hydrogen or halogen atoms; aliphatic, aromatic or heterocyclic groups; alkylamino or arylamino, or alkyloxy or aryloxy.

In an embodiment, a group of therapeutic compounds include those where, within the scope of the definitions of appended claim 1, R¹ is an alkyl, alkenyl, or aryl group, k is one, Z is a carbonyl group, R² is a hydrogen atom or an alkyl group, m is zero, p and s are 1, T is an alkylene group, and Y is SO₃X wherein X is H⁺ or another cation, such as alkali metal cations. In another embodiment a group of therapeutic compounds include those where, within the scope of the definitions of appended claim 1, R¹ and R² are alkyl, alkenyl, or aryl groups, or R¹ and R² are taken together to form an alkylene group, k and m are each one, Z and Q are carbonyl groups, p and s are 1, T is an alkylene group, and Y is SO₃X where X is H⁻ or another cation, such as alkali metal cations.

In another embodiment a group of therapeutic compounds include those where, within the scope of the definitions of appended claim 1, R¹ is an alkyl, alkenyl, or aryl group, k and m are zero, R³ is hydrogen or an alkyl group, p and s are each one, T is an alkylene group, and Y is SO₃X wherein X is H⁻ or another cation, such as alkali metal cations. In another embodiment, a group of therapeutic compounds include those where, within the scope of the definitions of appended claim 1, R¹ and R² are alkyl, alkenyl, or aryl groups, or R¹ and R² are taken together to form an alkylene group, k and m are zero, p and s are each one, T is an alkylene group, Y is SO₃X where X is H⁺ or another cation, such as alkali metal cations.

Not intending to be bound by theory, it is believed that under physiological conditions it is preferable that the nitrogen of the therapeutic compound is converted into an ammonium salt. In keeping with this theory, it is believed that acetylated nitrogens are hydrolyzed by an enzyme and conversed into a positively charged ammonium group under normal physiological conditions. Likewise, in cases where the amine nitrogen is dialkylated, it is believed that the nitrogen is converted into an ammonium group by enzymatic activity. It is further believed that these conversions better enable the therapeutic compounds of the invention to interact with amyloid aggregates and/or amyloid precursors, e.g., cross the blood brain barrier, cross membranes, solubilize, etc., under physiological conditions *in vivo.*

For purposes of the present disclosure, the anionic group is a sulfonate group Bioisosteres encompass both classical bioisosteric equivalents and non-classical bioisosteric equivalents. Classical and non-classical bioisosteres of sulfate and sulfonate groups are known in the art (see e.g. Silverman, R.B. The Organic Chemisury of Drug Design and Drug Action, Academic Press, Inc.: San Diego, CA, 1992, pp. 19-23). Accordingly, a therapeutic compound can comprise at least one anionic group including sulfonates, sulfates, sulfamates, phosphonates, phosphates, carboxylates, and heterocyclic groups of the following formulae:

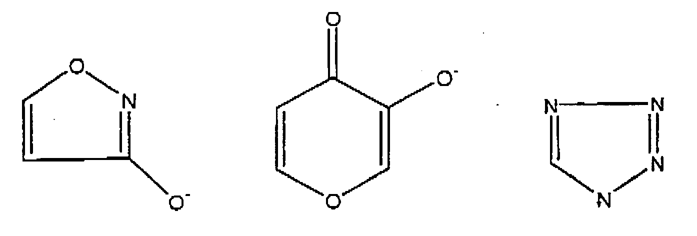

A therapeutic compound of the invention typically further comprises a counter cation (i.e., X⁻ in formula **(i))**. Cationic groups include positively charged atoms and moieties. If the cationic group is hydrogen, H⁻, then the compound is considered an acid, e.g., 3-acetylamino-1-propanesulfonic acid. If hydrogen is replaced by a metal or its equivalent, the compound is a salt of the acid. Pharmaceutically acceptable salts of the therapeutic compound are within the scope of the invention. For example, X⁻ can be a pharmaceutically acceptable alkali or alkaline earth metal, polycationic counter ion or ammonium. A preferred pharmaceutically acceptable salt is a sodium salt but other salts are also contemplated within their pharmaceutically acceptable range.

Within the therapeutic compound, the Y group(s) is covalently attached to a linking group T. Linking group T is of the formula -(CD¹D²)ₙ-, wherein n is an integer from 1 to 25, C is carbon and D¹ and D² are independently hydrogen or halogen atoms; aliphatic, aromatic or heterocyclic groups; alkylamino or arylamino; or alkyloxy or aryloxy.

The term "aliphatic group" is intended to include organic groups characterized by straight or branched chains, typically having between 1 and 22 carbon atoms. Aliphatic groups include alkyl groups, alkenyl groups and alkynyl groups. In complex structures, the chains can be branched or cross-linked. Alkyl groups include saturated hydrocarbons having one or more carbon atoms, including straight-chain alkyl groups and branched-chain alkyl groups. Such hydrocarbon moieties may be substituted on one or more carbons with, for example, a halogen, a hydroxyl, a thiol, an amino, an alkoxy, an alkylcarboxy, an alkylthio, or a nitro group. Unless the number of carbons is otherwise specified, "lower aliphatic" as used herein means an aliphatic group, as defined above (e.g., lower alkyl, lower alkenyl, lower alkynyl), but having from one to six carbon atoms. Representative of such lower aliphatic groups, e.g., lower alkyl groups, are methyl, ethyl, n-propyl, isopropyl, 2-chloropropyl, n-butyl, sec-butyl, 2-aminobutyl, isobutyl, tert-butyl, 3-thiopentyl, and the like. As used herein, the term "amino," means a -NH₂; the term "nitro" means -NO₂ ; the term "halogen" designates -F, -Cl, -Br or -I; the term "thiol" means a -SH; and the term "hydroxyl" means -OH. Thus, the term "alkylamino" as used herein means a -NHR, in which R is an alkyl group as defined above.. The term "alkylthio" refers to a -SR, in which R is an alkyl group as defined above. The term "alkylcarboxyl" means a -CO₂R, in which R is an alkyl group as defined above. The term "alkoxy" as used herein means a -OR, in which R is an alkyl group as defined above. Representative alkoxy groups include methoxy, ethoxy, propoxy, tert-butoxy and the like. The terms "alkenyl" and "alkynyl" refer to unsaturated aliphatic groups analogous to alkyls, but which contain at least one double or triple bond respectively.

The term "alicyclic group" is intended to include closed ring structures of three or more carbon atoms. Alicyclic groups include cycloparaffins or naphthenes which are saturated cyclic hydrocarbons, cycloolefins which are unsaturated with two or more double bonds, and cycloacetylenes which have a triple bond. They do not include aromatic groups. Examples of cycloparaffins include cyclopropane, cyclohexane, and cyclopentane. Examples of cycloolefins include cyclopentadiene and cyclooctatetraene. Alicyclic groups also include fused ring structures and substituted alicyclic groups such as alkyl substituted alicyclic groups. In the instance of the alicyclics such substituents can further comprise a lower alkyl, a lower alkenyl, a lower alkoxy, a lower alkylthio, a lower alkylamino, a lower alkylcarboxyl, a nitro, a hydroxyl, -CF₃, -CN, or the like.

The term "heterocyclic group" is intended to include closed ring structures in which one or more of the atoms in the ring is an element other than carbon, for example, nitrogen, or oxygen. Heterocyclic groups can be saturated or unsaturated and heterocyclic groups such as pyrrole and furan can have aromatic character. They include fused ring structures such as quinoline and isoquinoline. Other examples of heterocyclic groups include pyridine and purine. Heterocyclic groups can also be substituted at one or more constituent atoms with, for example, a halogen, a lower alkyl, a lower alkenyl, a lower alkoxy, a lower alkylthio, a lower alkylamino, a lower alkylcarboxyl, a nitro, a hydroxyl, -CF₃, -CN, or the like.

The term "aromatic group" is intended to include unsaturated cyclic hydrocarbons containing one or more rings. Aromatic groups include 5- and 6-membered single-ring groups which may include from zero to four heteroatoms, for example, benzene, pyrrole, furan, thiophene, imidazole, oxazole, thiazole, triazole, pyrazole, pyridine, pyrazine, pyridazine and pyrimidine, and the like. The aromatic ring may be substituted at one or more ring positions with, for example, a halogen, a lower alkyl, a lower alkenyl, a lower alkoxy, a lower alkylthio, a lower alkylamino, a lower alkylcarboxyl, a nitro, a hydroxyl, -CF₃, -CN, or the like.

The therapeutic compound of the invention can be administered in a pharmaceutically acceptable vehicle. As used herein "pharmaceutically acceptable vehicle" includes any and all solvents, dispersion media, coatings, antibacterial and antifungal agents, isotonic and absorption delaying agents, and the like which are compatible with the activity of the compound and are physiologically acceptable to the subject. An example of a pharmaceutically acceptable vehicle is buffered normal saline (0.15 molar NaCl). The use of such media and agents for pharmaceutically active substances is well known in the art. Except insofar as any conventional media or agent is incompatible with the therapeutic compound, use thereof in the compositions suitable for pharmaceutical administration is contemplated. Supplementary active compounds can also be incorporated into the compositions.

The therapeutic compound administered to the subject is of formula **(i)** above.

In an embodiment, "k" and "m" are both 0, and R¹ and R², taken together with the nitrogen to which they are attached, form an unsubstituted or substituted heterocycle, preferred groups include

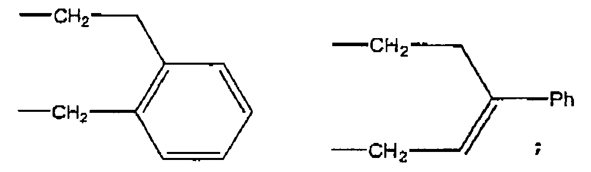

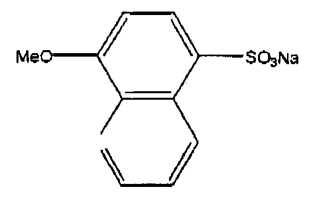

Preferred therapeutic compounds include 3-(3-hydroxy-1-propyl)amino-1-propanesulfonic acid **(LVX)**; 4-Phenyl-1-(3'-sulfopropyl)-1,2,3,6-tetrahydropyridine **(LVXV);** and 3-dimethylamino-1-propanesulfonic acid **(LVXVII),** and pharmaceutically acceptable salts thereof.

In an embodiment, therapeutic compounds include those where within the scope of the definitions of appended claim 1, R¹ is an alkyl, an alkenyl, or an aryl group, k is one, Z is a carbonyl group, R² is a hydrogen atom or an alkylene group, m is zero, p and s are 1, T is an alkylene group, and Y is SO₃X wherein X is H⁻ or another cation, such as alkali metal cations. Specific examples include mono-*N*-acylated compounds (e.g., R¹ is an alkyl, an alkenyl, or an aryl group, R² is a hydrogen atom or an alkyl group) such as 3-acetylamino-1-propanesulfonic acid **(VIII),** 2-acrylamido-2-methyl-1-propanesulfonic acid **(XXI),** and 3-benzoylamino-1-propancsulfonic acid **(X).** In another embodiment, a group of therapeutic compounds include those where, within the scope of the definitions of appended claim 1, R¹ and R² are alkyl, alkenyl, or aryl, or R¹ and R² are linked together to form an alkylene group, k and m are each one, Z and Q are each independently a carbonyl or a sulfonyl group, p and s are 1, T is an alkylene group, and Y is SO₃X where X is H⁻ or another cation, such as alkali metal cations. Specific examples include di-*N*-acylated compounds (including heterocyclic compounds, e.g., R¹ and R² are taken together to form an alkylene group) such as 3-phthalimido-1-propanesulfonic acid **(XXIII),** N-(3-sulfopropyl)saccharin sodium salt **(XXV),** and 4-phthalimido-1-butanesulfonic acid **(XIX).** In an advantageous embodiment, T is propylene or butylene.

In an embodiment a group of therapeutic compounds include those where, within the scope of the definitions of appended claim 1, R¹ is an alkyl, alkenyl, or aryl group, k and m are zero, R² is hydrogen or an alkyl group, or R¹ and R² are taken together to form an alkylene or an alkenylene group, p and s are each one, T is an alkylene group, and Y is SO₃X wherein X is H⁻ or another cation, such as alkali metal cations. Specific examples include mono-*N*-alkylated or arylated compounds such as 3-phenylamino-1-propanesulfonic acid sodium salt **(XIII),** 3-(4-pyridylamino)]-1-propanesulfonic acid **(XII),** 3-(benzylamino)-1-propanesulfonic acid **(XV),** 2-deoxy-2-(3-sulfopropyl)amino-d-glucose **(XX),** 1-phenyl-2,3,-dimethyl-4-methylamino-pyrazolon-5-*N*-methylsulfonic acid **(XXVII),** 3-[(-3,5-d-imethyl-1-adamantyl)-amino]-1-propanesulfonic acid **(XXIV),** 3-(2-hydroxyethyl)amino-1-propanesulfonic acid **(XXX),** 3-(3-hydroxy-1-propyl)amino-1-propanesulfonic acid **(XXXII),** (-)-3-[(*R*)-2-hydroxy-1-propyl]amino-1-propanesulfonic acid **(XXXIV),** 3-[(d,l)-1-hydroxy-2-propyl]-1-propanesulfonic acid **(XXXV),** 3-(4-hydroxy-1-butyl)amino-1-propanesulfonic acid **(XXXVI),** 3-(5-hydrox-1-pemyl)amino-1-propanesulfonic acid **(XXXI),** 3-(6-hydroxy-1-hexyl)amino-1-propanesulfonic acid **(XXXIII),** 3-(4-hydroxyphenyl)amino-1-propanesulfonic acid **(XL VI),** (+)-3-[(*S*)-2-hydroxy-1-propyl]amino-1-propanesulfonic acid **(XXXVII),** (+)-3-[(*S*)-1-hydroxy-2-propyl]amino-1-propanesulfonic acid **(XXXIX),** (-)-3-[(*R*)-1-hydroxy-2-propyl]amino-1-propanesulfonic acid **(XL),** (+)-3-[(*S*)-1-hydroxy-2-butyl]amino-1-propanesulfonic acid **(XLIII),** (-)-3-[(*R*)-1-hydroxy-2-butyl]amino-1-propanesulfonic acid **(XLIV),** 3-[(*dl*)-5-hydroxy-2-pentyl]amino-1-propanesulfonic acid **(XXXVIII),** 3-(*dl*)-6-hydroxy-2-hexyl]amino-1-propanesulfonic acid **(XLI),** 3-(1-hydroxymerhyl-1-cyclopentyl)amino-1-propanesulfonic acid **(XLII),** 3-amylamino-1-propanesulfonic acid **(XLV),** 3-hexylamino-1-propanesulfonic acid **(XLVII),** 3-heptylamino-1-propanesulfonic acid **(XLVIII),** 3-octylamino-1-propanesulfonic acid **(XLIX),** 3-nonylanuno-1-propanesulfonic acid **(L),** 3-decylamino-1-propanesulfonic acid **(LI),** 3-undecylamino-1-propanesulfonic acid **(LII),** 3-dodecylamino-1-propanesulfonic acid **(LIII),** 3-tridecylamino-1-propanesulfonic acid **(LIV),** 3-tetradecylamino-1-propanesulfonic acid **(LV),** 3-hexadecylamino-1-propanesulfonic acid **(LVI),** 3-octadecylamino-1-propanesulfonic acid **(LVII),** dimethyl(3-sulfopropyl)-tetradecylammonium hydroxide, inner salt **(LVXVIII),** and 2-(3-Sulfobutyl)-1,2,3,4-tenahydro-9H-pyrido [3,4-b] indole, sodium salt **(LVXIX).** In another embodiment a group of therapeutic compounds include those where, within the scope of the definitions of appended claim 1, R¹ and R² are alkyl, alkenyl, or aryl groups, or R¹ and R² are taken together to form an alkylene group, k and m are zero, p and s are each one, T is an alkylene group, Y is SO₃X where X is H⁻ or other cation such as cations of alkali metals. Specific examples include di-*N*-alkylated compounds (including heterocyclic compounds, e.g., R¹ and R² are alkylene) such as 3-dimethylamino-1-prapanesulfonic acid **(XI),** 4-(1-piperidinyl)-1-ethanesulfonic acid **(XIV),** 3-[1-(1,2,3,6-tetrahydropyridyl)]-1-propanesulfonic acid **(XVI),** 3-[2-(1,2,3,4-tetrahydroisoquinolinyl)]-1-propanesulfonic acid **(XVII),** 3-[2-(6,7-dimethoxy-1,2,3,4-tenahydroisoquinolinyl)]-1-propanesulfonie acid **(I),** 3-[1-(1,2,3,4-tetrahydroquinolinyl)]-1-propanesulfonic acid **(III),** 2-(3-sulfopropyl)-1,2,3,4-tetrahydro-9*H*-pyrido[3,4-*b*]indole, sodium salt **(V),** 3-(1-indolinyl)-1-propanesulfonic acid **(VII),** 3-[2-(6-methoxy-1,2,3,4-tetrahydrolsoquinolinyl)]-1-propanesulfonic acid **(IX),** 3-(2-isoindolinyl)-1-propanesulfonic acid **(II),** 2-(3-sulfopropyl)-(*S*)-nicotinium hydroxide inner salt **(IV),** 3-(4-benzyl-1-piperidinyl)-1-propanesulfonic acid **(VI),** 3-[2-(1,2,3,4.5,6,7,8-octahydroisoquinolinyl)]-1-propanesulfonic acid **(XVIII),** and [2-(1,2,3,4-tetrahydroisoquinolinyl)]-1-butanesulfonic acid **(XXVI)**.

R¹ may be a lower alkyl group, R² a lower alkyl group and T a lower alkylene group. Preferably, R¹ is a methyl, ethyl, or propyl group, R² is a methyl, ethyl or propyl group and T is an ethylene, propylene or butylene group.

The linking group may be substituted, e.g., with one or more amino, nitro, halogen, thiol or hydroxy groups.

A further aspect of the invention includes pharmaceutical compositions for treating amyloidosis. The therapeutic compounds of the invention, as described hereinbefore, can be incorporated into a pharmaceutical composition in an amount effective to modulate amyloidosis in a pharmaceutically acceptable vehicle.

Prodrugs are converted *in vivo* to the therapeutic compounds of the invention (see, e.g., R.B. Silverman, 1992, "The Organic Chemistry of Drug Design and Drug Action", Academic Press, Chp. 8). Such prodrugs can be used to alter the biodistribution (e.g., to allow compounds which would not typically cross the blood-brain barrier to cross the blood-brain barrier) or the pharmacokinetics of the therapeutic compound. For example, a sulfonate, can be esterified, e.g., with a methyl group or a phenyl group, to yield a sulfonate ester. When the sulfonate ester is administered to a subject, the ester is cleaved, enzymatically or non-enzymatically, reductively or hydrolyrically, to reveal the anionic group. Such an ester can be cyclic, e.g., a cyclic sultone, or two or more anionic moieties may be esterified through a linking group. In a preferred embodiment, the prodrug is a cyclic sultone. An anionic group can be esterified with moieties (e.g., acyloxymethyl esters) which are cleaved to reveal an intermediate compound which subsequently decomposes to yield the active compound. In another embodiment, the prodrug is a reduced form of a sulfonate, e.g., a thiol, which is oxidized *in vivo* to the therapeutic compound. Furthermore, an anionic moiety can be esterified to a group which is actively transported *in vivo*, or which is selectively taken up by target organs. The ester can be selected to allow specific targeting of the therapeutic moieties to particular organs, as described in more derail below

Within the therapeutic compound, the Y group(s) is covalently attached to a linking group T. Linking group T is of the formula -(CD¹D²)ₙ-, wherein n is an integer from 1 to 25, C is carbon and D¹ and D² are independently hydrogen or halogen atoms; aliphatic, aromatic or heterocyclic groups; alkylamino or arylamino; or alkyloxy or aryloxy.

Amyloid aggregation in a subject may be modulated by administering a therapeutic compound of the invention to a subject, i.e., *in vivo.* The term "subject" includes living organisms in which amyloidosis can occur. Examples of subjects include humans, monkeys, cows, sheep, goats, dogs, cats, mice, rats, and transgenic species thereof. Administration of the compositions of the present invention to a subject to be treated can be carried out using known procedures, at dosages and for periods of time effective to modulate amyloid aggregation in the subject. An effective amount of the therapeutic compound necessary to achieve a therapeutic effect may vary according to factors such as the amount of amyloid already deposited at the clinical site in the subject, the age, sex, and weight of the subject, and the ability of the therapeutic compound to modulate amyloid aggregation in the subject. Dosage regimens can be adjusted to provide the optimum therapeutic response. For example, several divided doses may be administered daily or the dose may be proportionally reduced as indicated by the exigencies of the therapeutic situation. A non-limiting example of an effective dose range for a therapeutic compound of the invention (e.g., 3-acetylamino-1-propylsulfonic acid, sodium salt) is between 5 and 500 mg/kg of body weight/per day. In an aqueous composition, preferred concentrations for the active compound (i.e., the therapeutic compound that can modulate amyloid aggregation) are between 5 and 500 mM, more preferably between 10 and 100 mM, and still more preferably between 20 and 50 mM. For *N*-acetylated homotaurine derivatives, particularly preferred aqueous concentrations are between 10 and 20 mM.

The therapeutic compounds of the invention are effective when administered orally. Accordingly, a preferred route of administration is oral administration. Alternatively, the active compound may be administered by other suitable routes such as subcutaneous, intravenous, intraperitoneal, etc. administration (e.g., by injection). Depending on the route of administration, the active compound may be coated in a material to protect the compound from the action of acids and other natural conditions which may inactivate the compound.

The compounds of the invention can be formulated to ensure proper distribution *in vivo.* For example, the blood-brain barrier (BBB) excludes many highly hydrophilic compounds. To ensure that the therapeutic compounds of the invention cross the BBB, they can be formulated, for example, in liposomes. For methods of manufacturing liposomes, see, e.g., U.S. Patents 4,522,811; 5,374,548; and 5,399,331. The liposomes may comprise one or more moieties which are selectively transported into specific cells or organs ("targeting moieties"), thus providing targeted drug delivery (see, e.g., V.V. Ranade (1989) J. Clin. Pharmacol. 29:685). Exemplary targeting moieties include folate or biotin (see, e.g., U.S. Patent 5,416,016 to Low et al.); mannosides (Umezawa et al., (1988) Biochem. Biophys. Res. Commun. 153:1038); antibodies (P.G. Bloeman et al. (1995) FEBS Lett. 357:140; M. Owais et a/. (1995) Antimicrob. Agents Chemother. 39:180); surfactant protein A receptor (Briscoe et al. (1995) Am. J. Physiol. 1233:134); gp120 (Schreier et al. (1994) J. Biol. Chem. 269:9090); see also K. Keinanen; M.L. Laukkanen (1994) FEBS Lett. 346:123; J.J. Killion; I.J. Fidler (1994) Immunomethods 4:273. In a preferred embodiment, the therapeutic compounds of the invention are formulated in liposomes; in a more preferred embodiment, the liposomes include a targeting moiety.

To administer the therapeutic compound by other than parenteral administration, it may be necessary to coat the compound with, or co-administer the compound with, a material to prevent its inactivation. For example, the therapeutic compound may be administered to a subject in an appropriate carrier, for example, liposomes, or a diluent. Pharmaceutically acceptable diluents include saline and aqueous buffer solutions. Liposomes include water-in-oil-in-water CGF emulsions as well as conventional liposomes (Strejan et al., (1984) J. Neuroimmunol. 7:27).

The therapeutic compound may also be administered parenterally, intraperitoneally, intraspinally, or intracerebrally. Dispersions can be prepared in glycerol, liquid polyethylene glycols, and mixtures thereof and in oils. Under ordinary conditions of storage and use, these preparations may contain a preservative to prevent the growth of microorganisms.

Pharmaceutical compositions suitable for injectable use include sterile aqueous solutions (where water soluble) or dispersions and sterile powders for the extemporaneous preparation of sterile injectable solutions or dispersion. In all cases, the composition must be sterile and must be fluid to the extent that easy syringability exists. It must be stable under the conditions of manufacture and storage and must be preserved against the contaminating action of microorganisms such as bacteria and fungi. The vehicle can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, sodium chloride, or polyalcohols such as mannitol and sorbitol, in the composition. Prolonged absorption of the injectable compositions can be brought about by including in the composition an agent which delays absorption, for example, aluminum monostearate or gelatin.

Sterile injectable solutions can be prepared by incorporating the therapeutic compound in the required amount in an appropriate solvent with one or a combination of ingredients enumerated above, as required, followed by filtered sterilization. Generally, dispersions are prepared by incorporating the therapeutic compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient (i.e., the therapeutic compound) plus any additional desired ingredient from a previously sterile-filtered solution thereof.

The therapeutic compound can be orally administered, for example, with an inert diluent or an assimilable edible carrier. The therapeutic compound and other ingredients may also be enclosed in a hard or soft shell gelatin capsule, compressed into tablets, or incorporated directly into the subject's diet. For oral therapeutic administration, the therapeutic compound may be incorporated with excipients and used in the form of ingestible tablets, buccal tablets, troches, capsules, elixirs, suspensions, syrups, wafers, and the like. The percentage of the therapeutic compound in the compositions and preparations may, of course, be varied. The amount of the therapeutic compound in such therapeutically useful compositions is such that a suitable dosage will be obtained.

It is especially advantageous to formulate parenteral compositions in dosage unit form for ease of administration and uniformity of dosage. Dosage unit form as used herein refers to physically discrete units suited as unitary dosages for the subjects to be treated; each unit containing a predetermined quantity of therapeutic compound calculated to produce the desired therapeutic effect in association with the required pharmaceutical vehicle. The specification for the dosage unit forms of the invention are dictated by and directly dependent on (a) the unique characteristics of the therapeutic compound and the particular therapeutic effect to be achieved, and (b) the limitations inherent in the art of compounding such a therapeutic compound for the treatment of amyloid aggregation in subjects.

Active compounds are administered at a therapeutically effective dosage sufficient to modulate amyloid aggregation in a subject. A "therapeutically effective dosage" preferably modulates amyloid aggregation by at least about 20%, more preferably by at least about 40%, even more preferably by at least about 60%, and still more preferably by at least about 80% relative to untreated subjects. The ability of a compound to modulate amyloid aggregation can be evaluated in model systems that may be predictive of efficacy in modulating amyloid solubility and aggregation in human diseases, such as animal model systems known in the art or by *in vitro* methods including the thioflavine T assay, circular dichroism and electron microscopy. Other *in vitro* methods can be used to determine the ability of a compound to bind to the soluble amyloidogenic protein and keep it soluble, such as equilibrium dialysis, NMR and solubilization assays. Methods where adherence of soluble or non-soluble (e.g., fibrillary) amyloid protein to cell surface is monitored or determined include immunodetection of the protein at the cell surface, light microscopy, electron microscopy and flow cytometry.

The compound of the invention is useful for treating amyloidosis associated with any disease in which amyloid aggregation occurs. Clinically, amyloidosis can be primary, secondary, familial or isolated. Amyloids have been categorized by the type of amyloidogenic protein contained within the amyloid. Non-limiting examples of amyloids which can be modulated, as identified by their amyloidogenic protein, are as follows (with the associated disease in parentheses after the amyloidogenic protein): β-amyloid (Alzheimer's disease, Down's syndrome, hereditary cerebral hemorrhage amyloidosis [Dutch]); amyloid A (reactive [secondary] amyloidosis, familial Mediterranean Fever, familial amyloid nephropathy with urticaria and deafness [Muckle-Wells syndrome]); amyloid κ L-chain or amyloid λ L-chain (idiopathic [primary], myeloma or macroglobulinemia-associated); Ab2M (chronic hemodialysis); ATTR (familial amyloid polyneuropathy [Portuguese, Japanese, Swedish], familial amyloid cardiomyopathy [Danish], isolated cardiac amyloid, systemic senile amyloidosis); AIAPP or amylin (adult onset diabetes, insulinoma); atrial naturetic factor (isolated atrial amyloid); procalcitonin (medullary carcinoma of the thyroid); gelsolin (familial amyloidosis [Finnish]); cystatin C (hereditary cerebral hemorrhage with amyloidosis [Icelandic]); AApoA-I (familial amyloidotic polyneuropathy [Iowa]); AApoA-II (accelerated senescence in mice); fibrinogen-associated amyloid; lysozyme-associated amyloid; and AScr or PrP-27 (Scrapie, Creutzfeldt-Jacob disease, Gerstmann-Straussler-Scheinker syndrome, bovine spongiform encephalitis, and TSE).

The ability of a compound to modulate amyloid aggregation can be evaluated in an animal model system that may be predictive of efficacy in inhibiting amyloid aggregation in human diseases. The ability of a compound to inhibit amyloid aggregation can also be evaluated by examining the ability of the compound to inhibit amyloid aggregation *in vitro* or *ex vivo*, e.g., using an ELISA assay. The effect of a compound on the secondary structure of the amyloid can be further determined by circular dichroism (CD), infrared (IR) spectroscopy, and electron microscopy.

CD and IR spectroscopy are particularly useful techniques because the information obtained is a direct measure of the ability of a test compound to maintain the amyloid proteins in soluble non β-sheet form, by determining the structural effect of a compound on amyloid protein folding and/or fibril formation. This contrasts with previously known methods which measure cellular trafficking of amyloid protein precursors or interactions between amyloid and extracellular matrix proteins, providing only indirect evidence of potential amyloid-inhibiting activity. It should further be noted that CD and IR spectroscopy can also detect compounds which cause an increase in, e.g., β-sheet folding of amyloid protein, and thereby stabilize the formation of amyloid fibrils. Electron microscopy can be used to visualize directly the ability of a compound to maintain the amyloid protein in a soluble non-fibrillar state.

The aggregation of amyloid is a multi-stage process. Accordingly, an agent useful for treating amyloidosis has many potential modes of action. An agent which inhibits amyloid aggregation, and the related cellular toxic effect , could act in one or more of the following ways, which are shown by way of illustration and not limitation:
1. Inhibition or delay of protein assembly or oligomerization in solution
2. Inhibition or delay of aggregation of amyloid assemblies or oligomers into insoluble β-sheet structures and/or aggregates
3. Disruption/dissolution/modification of insoluble amyloid fibrils and/or aggregates
4. Inhibition of the soluble or fibrillar amyloid protein binding to the cell surface, leading to a cellular activation process or toxicity.

Categories 1 and 2 correspond to prevention of the formation of amyloid aggregates (slowing down or halting amyloid aggregation), and category 3 corresponds to removal or modification of aggregates already formed (removal or reduction of existing amyloid aggregates). Category 4 focuses on the inhibition of the amyloid protein interaction in the cell surface.

The invention is further illustrated by the following examples which should not be construed as further limiting the subject invention.

### EXAMPLE 1

A solubility assay using Bradford detection was conducted to demonstrate the activity of certain therapeutic compounds in preventing or inhibiting Aβ fibril formation accordance with the present disclosure. Aβ peptides were synthesized using standard FMOC chemistry which was performed in conjunction with the Biotechnology Centre, University of Toronto, and purified by HPLC. Alternatively, peptides can also be obtained from a number of commercial sources (e.g., BaChem and Peninsula Laboratories, California).

The assay was conducted as follows. Stock solution of Aβ42 or Aβ40 peptide at 5 mg/ml in distilled water, pH 7, and stock solutions of each test compound at 2 mg/ml in distilled water, pH 7 were prepared.
1. Mix 5µl (25µg) of stock Aβ and 12.5 µl (25 µg) of stock test compounds into 1000 µl of 10 mM phosphate buffer, pH 7. This provides a molar ratio of roughly 1:10 [peptide:compound] assuming a general molecular weight of 400 daltons for the test compounds. Control samples were prepared for both peptide and compound. These contained Aβ only using both 25 µg (5 µl stock Aβ) and 50 µg (10µl stock Aβ) to provide a standard curve for each run. Test compound controls contained 25 µg of material (12.5 µl of stock). All samples were mixed in 1000 µl of 10 mM phosphate buffer, pH 7 to final volume of 1017.5 µl.
2. Incubate all samples overnight at room temperature without mixing.
3. Spin at 14,000 rpm for 10 min in table top Eppendorf microfuge.
4. Take 800 µl of supernatant.
5. Add 200 µl of Bradford reagent (purchased from BioRad).
6. Mix well by vortexing.
7. Read at OD595 nm.

Compounds were characterized as "moderately active" if 25-50% of A•42 remained in the supernatant, "active" if 50-75% of Aβ42 remained.

| Active | Very active |
|---|---|
| **XVII** | **XXII** (Comparative Compound) |
| | **XXIX** (Comparative Compound) |

### EXAMPLE 2

An ELISA solubility assay was conducted to demonstrate the activity of certain therapeutic compounds in preventing or inhibiting Aβ fibril formation accordance with the present disclosure.

The assay was conducted as follows. Stock solution of Aβ42 peptide at 5 mg/ml in distilled water, pH 7, and stock solutions of each test compound at 1 mg/ml in distilled water, pH 7 were prepared.
1. A 10 µg sample of peptide was mixed with compound at a molar ratio of 1:10 [pepride:compound] in 500µl of 10 mM phosphate buffer. Control samples contained peptide only,
2. The mixture was incubated overnight at room temperature without agitation.
3. The incubated mixture was centrifuged at 14,000 rpm (Eppendorf microfuge) for 5 minutes to separate the soluble peptide.
4. 400 µl aliquots of supernatant were removed for ELISA assay.

### ELISA

1. 100 µl of prepared samples were coated in 96 wells NUNC microplates (each sample tested in triplicate)
2. The plate was incubated at 37 °C for 3 hours, then at 4 °C overnight.
3. The wells were washed twice with 0.05% Tween 20 in phosphate buffered saline.
4. 250 µl of 3% skim milk powder in PBS was used to block non-specific binding to the wells (at 37 °C for 1.5 hours)
5. The wells were washed with 0.05% Tween 20 / PBS twice.
6. A 100 µl aliquot of diluted (final dilution 1:100 in PBS) mouse monoclonal anti-Aβ antibody (purchased from DAKO recognizing the N-terminal residues 1-10) was added to each well. The antibody was then incubated at 37 C for 2 Hours.
7. The wells were washed with 0.05% Tween 20/ PBS six times (5 min/wash).
8. Visualization was performed using 100 µl of diluted goat anti-mouse IgG (H+L) conjugated with alkaline phosphatase (purchased from BioRad) was added to each well. The plate was incubated at 37 °C for 1 hour.
9. The wells were washed with 0.05% Tween 20 / PBS six times (5 min/wash).
10. The color reaction was developed using 100 µl of alkaline phosphatase substrate (purchased from BioRad) which was added to each well.
11. The relative amounts of Aβ were obtained by measuring the OD of the sample at 405 nm using a standard ELISA plate reader.

Compounds were characterized as "active" if 40-50% of Aβ42 remained in the supernatant.

| Active |
|---|
| **XXIV** |
| **XXVIII** (Comparative Compound) |
| **XXI** |

### EXAMPLE 3

Circular dichroism analysis was conducted to demonstrate the activity of certain therapeutic compounds in preventing or inhibiting Aβ40 fibril formation accordance with the present disclosure by determining the presence or absence of β-sheet conformation.

The assay is conducted as follows:

### INSTRUMENT AND PARAMETERS

Instrument: JASCO J-715 Spectropolarimeter.
Cell/cuvette: Hellma quartz (QS) with 1.0 mm pathlength
Room temperature.
Wavelength interval: 250 nm-190 nm.
Resolution: 0.1 nm.
Band width: 1.0 nm
Response time: 1 sec
Scanning speed: 20 nm/min
Number of accumulations/spectrum: 5

### PRE-INCUBATION ASSAY

1. Prepare a fresh 40µm solution of Aβ(1-40) in 0.02M Tris, pH 7.4.
2. Prepare a 1mM solution of test compounds in 0.02M Tris, pH 7.4.
3. Combine equal volumes of the Aβ(1-40) and test compound solutions.
4. Incubate the mixtures for 19h.
5. Take the CD spectrum using the parameters above.
6. Return the mixtures to the incubator and incubate to 43h.
7. Take the CD spectrum using the parameters above.

Inhibition of Aβ40 assembly/aggregation is determined by comparing the amount of b-sheet structure )appearing at lambda=218nm) obtained in control and in treated sample at each timepoint.

| **Compound** | **Incubation time** | | |
|---|---|---|---|
| | 0h | 19h | 43h |
| **XVII** | -^{1,2} | + | ++ |
| **III** | - | + | ++ |
| **VII** | + | ++ | ++ |
| **IX** | + | +++ | ++ |
| **VIII** | + | ++++ | +++ |
| **X** | + | ++ | ++ |
| **XXII** (Comparative compound) | + | ++++ | ++ |
| **LVXIII** (Comparative compound) | - | ++++ | - |
| **LVXV** | + | ++++ | ++ |
| **LVXVI** (Comparative compound) | + | +++ | ++ |
| **XXXVIII** | - | +++ | ++ |

| | | | |
|---|---|---|---|
| ¹No effect compared to Aβ alone ²Key: relative inhibition compared to Aβ alone : +: 0-25%; ++: 25-50%; +++: 50-75%; ++++: 75-100% | | | |

### EXAMPLE 4

CD analysis was conducied as above to demonstrate the activity of certain therapeutic compounds in preventing or inhibiting IAPP fibril formation.

| Compound | Activity |
|---|---|
| **XLIII** | - |
| **XXXVIII** | Active |
| **XLII** | Active |

### EXAMPLE 5

Secondary amyloidosis *in vivo* results.

The *in vivo* screening is based on the acute AEF-AgNO₃-induced amyloidosis mouse model. The test is conducted on a total of 6 days and each compound administered in the drinking solution for a 5-day period.

Female mice of CBA/J strain are individually identified, weighed and assigned to a group of 5 animals following an acclimation period. The amyloidosis is induced by intravenous injection of 100 µg of AEF (amyloid enhancing factor) concomitantly with a subcutaneous injection of 0.5 ml of 2% of a solution of AgNO₃. Animals in the negative control group are injected with saline only.

Twenty-four hours following the amyloidosis induction, the compound is added to a 1% solution of sucrose (vehicle) and distributed to animals in drinking bottles for a period of 5 days. Animals in the positive control group receive the vehicle only. Drinking solutions are made available *ad libitum* to each group of animals and the volume measured before and after use to calculate the consumption of each solution. Blood samples are collected for *in vitro* determination of plasma serum amyloid A levels.

On day 6, mice are sacrificed, weighed and organs such as spleen are existed and fixed in acid alcohol. Spleen samples are processed, embedded in paraffin wax and cut inyo sections. Spleen sections from each animal are stained with the Congo Red staining solution and the splenic Amyloid A fibrils aggregation evaluated by image analysis. Results are expressed as % of AA fibrils deposited in the splenic perifollicular area. Raw data is analyzed.

Mean of mouse body weight and the mean consumption of the drinking solution (ml) are compiled. Variation of the body weight before the induction and at the end of the assay is calculated (%). The dose level of the compound consumed (mg/kg/day) is calculated as well.

P-values for Student t-test and Mann-Whitney tests comparing a group to the positive control are calculated using GraphPad Prism computer software.

The mean of the image analysis reading of spleen serious for a group is expressed as percentage of the mean of the image analysis readings for the positive control group (%PC).

| Compound | Concentration (mg/ml) | %PC | Activity |
|---|---|---|---|
| **I** | 6.25 | 87 | Moderate |
| | 12.5 | 76%, 68% | |
| | 25.0 | 69% | |
| **III** | 3.5 | 113% | Moderate |
| | 6.25 | 87% | |
| | 12.5 | 54%, 67%, 56% | |
| | 25.0 | 82%, 77% | |
| **VII** | 6.25 | 94%, 91% | Moderate |
| | 12.5 | 83% | |
| | 25.0 | 71%, 97% | |
| **IX** | 6.25 | 74%, 40% | Active |
| | 12.5 | 64%, 38% | |
| **XVIII** | 3.13 | 74% | Moderate |
| | 6.25 | 86% | |
| **XX** | 6.25 | 94% | Moderate |
| | 12.5 | 69% | |

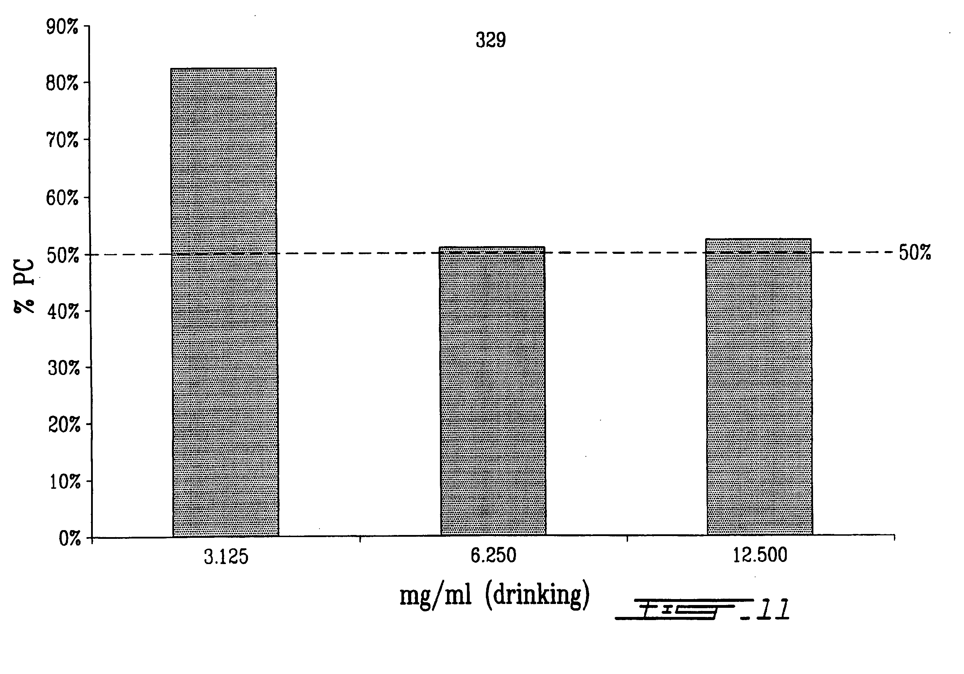

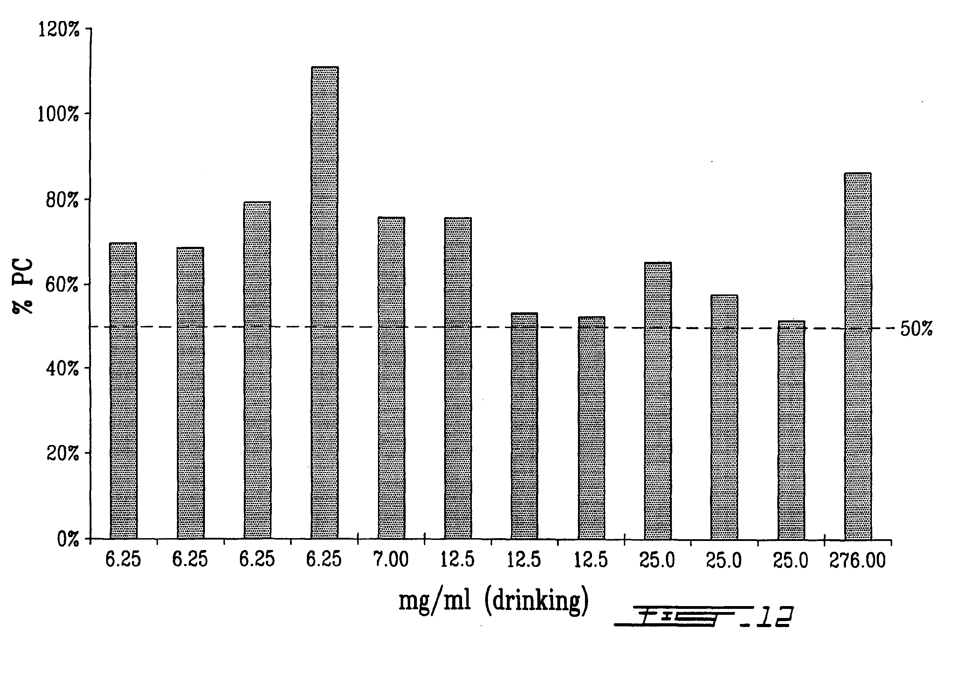

The effectiveness of compounds of the invention, **XXVII** and **XVII** are shown in Figures 11 and 12, which are histograms showing results using the above animal model.

### EXAMPLE 6

### Determination of the rate of amyloid fibril formation by Thioflavine T spectroscopy

Thioflavine T (ThT) binds to amyloid proteins in β-sheet formation, exhibiting a yellow fluorescence from tissue sections and fibrils *in vitro.* Detection of ThT fluorescence can be used as a sensitive assay for amyloid fibril formation under different conditions. This assay has been used in experiments to determine the effects of compounds of the invention on amyloid fibril formation.

### Method

Human IAPP was dissolved in 40% trifluoroethanol and freeze-dried into conveniently-sized aliquots. IAPP was prepared immediately before the measurements by dissolving in 40% 1,1,1,3,3,3-hexafluoro-2-propanol (HFIP) in water to maintain the peptide in alpha helical conformation and soluble. A stock solution of ThT (2.5mM) was prepared, 7.9mg in 10mL Tris-HCl pH 7.0 and filtered (0.22 µm). Solutions were kept in the dark until use. Fluorescence was examined at 440nm excitation (slit 5nm), and emission at 482nm (slit 10nm) with stirring. 25ml of ThT stock (final concentration 62.5 µM) was added to peptide sample and made up to 1mL in the cuvette. The sample was stirred for 5 min. before taking a reading. Measurements were made at an initial time point (5 min. from sample preparation), at intervals over the next 4-6h and after overnight incubation at room temperature.

Certain compounds as disclosed herein, i.e., 3-(3-hydroxy-1-propyl)amino-1-propanesulfonic acid; DL-2-amino-5-phosphovaleric acid; 4-Phenyl-1-(3'-sulfopropyl)-1,2,3,6-tetrahydropyridine; cyclohexylsulfamic acid; *O*-phospho-*L-*serine; 8-methoxyquinoline-5-sulfonic acid; 3-amino-2-hydroxy-1-propanesulfonic acid; and 3-dimethylamino-1-propanesulfonic acid, and 1,2,3,4-tetrahydroisoquinoline, were found to inhibit or prevent IAPP-associated fibril assembly.

### EXAMPLE 7

Circular dichroism analysis was conducted to confirm the activity of certain therapeutic compounds in preventing or inhibiting IAPP-associated fibril formation in accordance with the present disclosure by determining the presence or absence of β-sheet conformation.

The assay is conducted as follows:

### INSTRUMENT AND PARAMETERS

Instrument: JASCO J-715 Spectropolarimeter
Cell/cuvette: Hellma quartz (QS) with 1.0 mm pathlength
Room temperature
Wavelength interval: 250 nm-190 nm
Resolution: 0.1 nm
Band width: 1.0 nm
Response time: 1 sec
Scanning speed: 20 nm/min
Number of spectra run: 5

The assay, a co-incubation procedure, examines the ability of a compound or substance to inhibit the assembly of amyloid fibrils, e.g., to test for the presence of the amyloidotic β-sheet conformation in the presence of soluble IAPP. Samples are run in the presence and absence (i.e., water alone) of buffering agent, which is done to determine if competitive effects are seen with the ionic buffer (usually phosphate).

### A. Assay in Water Only

Add components used at a molar ratio of 1:10 [peptide:compound]; add 10 µL of 10 mg/mL IAPP stock solution (final 100 µg peptide) to the aqueous solution containing compound to a final volume of 400 µl. The pH of the final assay solution is measured to ensure there is no fluctuation and the spectrum is accumulated using the parameters as shown above.

### B. Assay in Phosphate Buffer

Add desired amount of compound to achieve a 1:10 molar ratio in 10 mM phosphate buffer, pH 7. Add 10 µL of 10 mg/mL IAPP stock solution (final peptide 100 µg) to the phosphate buffered solution containing the compound and bring to a final volume of 400 µL. The pH of the final assay solution is measured to ensure there is no fluctuation and the spectrum is accumulated using the parameters as shown above.

In both assays, a control sample is run with each test group. This control contains peptide only in water or buffer at a similar final volume of 400 µl. Spectra for the control are collected initially (first run) and at the end of the test (final run) to ensure that the peptide has not undergone extensive aggregation during the course of the assay. Spectra for the controls are used to compare with the measurements obtained with the treated samples.

### CO-INCUBATION:

Make fresh 1 mg/mL stock solution of IAPP in 10 mM phosphate buffer, pH 7. Add desired amount of compound to achieve a 1:10 molar ratio in 10 mM phosphate buffer, pH 7. Incubate for 3 days at room temperature. Make up to final volume of 400 µL with 10 mM phosphate buffer, pH 7. The pH of the final assay solution is measured to ensure there is no fluctuation and the spectrum is accumulated using the parameters as shown above.

A similar control is run for comparative purposes.

### DATA ANALYSIS

Plots of the spectra (control and treated) are individually assembled and the changes in ellipticity at 218 nm are examined. This minimum is directly correlated with the amount of β-sheet present in the sample. Changes in either a positive or negative direction are noted and a relative value ("active" or "not active") assigned to the compound as a measure of activity. In a subsequent experiment with the compounds at a molar ratio of 1:5 [peptide:compound], the degree of randomness was noted, an indication of the ability of the compounds to prevent amyloid aggregation. A more positive number indicates less β-sheet formation. The ability of a compound to prevent β-sheet formation for at least 24h is important, as the non-aggregated amyloid fibrils will be excreted in the soluble form. In the control noted below, the decrease in CD (mdegs) may indicate that some of the peptide is aggregating under these conditions.

| Compound | Activity | T₀ | 24h | 48h |
|---|---|---|---|---|
| Control IAPP | - | Random | β (-2) | β (-1.5) |
| 3-(3-hydroxy-1-propyl)amino-1-propanesulfonic acid **(LVX)** | Active | Random | Random | β (-1.7) |
| DL-2-amino-5-phosphovaleric acid **(LVIII)** | Active | Random | Random | β (-3.5) |
| 1,2,3,4-tetrahydrolsoquinoline(**LVIX**) | Active | Random | β (-1.5) | β (-1.3) |
| cyclohexylsulfamic acid(**LVXI**) (Comparative compound) | Active | Random | β (-1.1) | β (-0.8) |
| O-phospho-L-serine (**LVXII**) (Comparative compound) | Active | Random | Random | β (-2.0) |
| 8-methoxyquinoline-5-sulfonic (Comparative compound) acid(**LVXIV**) | Active | Random | β (-1.3) | β (-0.8) |
| 4-Phenyl-1-(3'-sulfopropyl)-1,2,3,6-tetrahydropyridine, sodium salt (**LVXV**) | Active | Random | Random | β (-1.8) |
| 3-amino-2-hydroxy-1-propanesulfonic acid (**LVXVI**) (Comparative compound) | Active | - | - | - |
| 3-dimethylamino-1-propancsulfonic acid (**LVXVII**) | Active | Random | β (-1.7) | β (-1.5) |

### EXAMPLE 8

The synthesis of a compound of the invention, 4-Phenyl-1-(3'-sulfopropyl)-1,2,3,6-tetrahydropyridine, is described below.

To a solution of 4-phenylpyridine (15.5 g, 0.1 mol) in acetone (100 mL) was added 1,3-propane sultone (12.2 g, 0.1 mol) at room temperature. The mixture was then heated at reflux temperature overnight. The resultant suspension was cooled to room temperature. The solid was collected by filtration and washed with acetone. To a solution of the solid (31 g) in methanol (500 mL) was added sodium borohydride (10 g, 260 mmol) portionwise, and the mixture was stirred at room temperature for 2 h. Distilled water (50 mL) was added to destroy the excess of sodium borohydride. The mixture was diluted with methanol (200 ml), and neutralized with Amberlite IR-120 ion-exchange resin (H⁺ form, 300 g). A white precipitate was formed. The precipitate and the resin were removed by filtration and treated with distilled water (400 mL) at ∼100 °C. The mixture was filtered and the residual resin was washed with hot distilled water (2 x 200 mL). The filtrates and washings were combined and concentrated to dryness. The residue was co-evaporated with methanol (3 x 200 mL), and then recrystallized from ethanol-water {8:2 (v/v)} to afford 4-phenyl-1-(3'-sulfopropyl)-1,2,3,6-tetrahydropyridine as white crystals (26 g, 93%). The ¹H and ¹³C NMR spectra were in agreement with the structure.

To a solution of 4-phenyl-1-(3'-sulfopropyl)-1,2,3,6-tetrahydropyridine (5.6 g, 20 mmol) obtained above in ethanol (180 mL) was added sodium hydroxide (1.2 g, 30 mmol). The suspension was heated at reflux temperature for 30 min. The mixture was then cooled to room temperature. The first crop of product (3.9 g, 64%) was collected by filtration. The filtrate was concentrated to dryness, and the residue was recrystallized from ethanol to afford the second crop of product (2.0 g, 32%). ¹H NMR (400 MHz, D₂O): δ 1.85 (quintet, 2 H, J 8.7, 7.7 Hz, 2 H-2'), 2.39-2.45 (m, 4 H, 2 H-3' and 2 H-3), 2.59 (t, 2 H, J 5.6 Hz, 2 H-2), 2.80 (t, 2 H, J 7.7 Hz, 2 H-1'), 3.00 (br s, 2 H, 2 H-6), 6.00 (br s, 1H, H-5), 7.18-7.36 (m, 5 H, Ar). ¹³C NMR (100.6 MHz, D₂O): δ 23.90 (C-2'), 29.01 (C-3), 51.69, 51.76 (C-2, C-3'), 54.45 (C-6), 58.12 (C-1'), 123.75 (C-5), 127.31, 130.01, 131.24 (Ar), 136.89 (C-4), 142.47 (Ar).

### EXAMPLE 9

The synthesis of a comparative compound, 8-methoxy-5-quinolinesulfonic acid, sodium salt, is described below.

8-methoxy-5-quinoline (3.8 g. sublimated) was added to cold chlorosulfonic acid (30 mL at 2°C) over 30 min. The reaction mixture was stirred at room temperature (ca. 20°C) for 1h. TLC showed complete consumption of starting material at this time. The reaction mixture was poured onto ice (200 g), and sodium carbonate (70 g) was then added. The solid material was collected by filtration and then dissolved in ethyl acetate (250 mL) which was then washed with water. The organic layer was then separated and (Na₂CO₃). The organic layer was then filtered and the solvent was evaporated *in vacuo* to yield 8-methoxy-5-quinolinesulfonyl chloride as a white solid (2.9g).

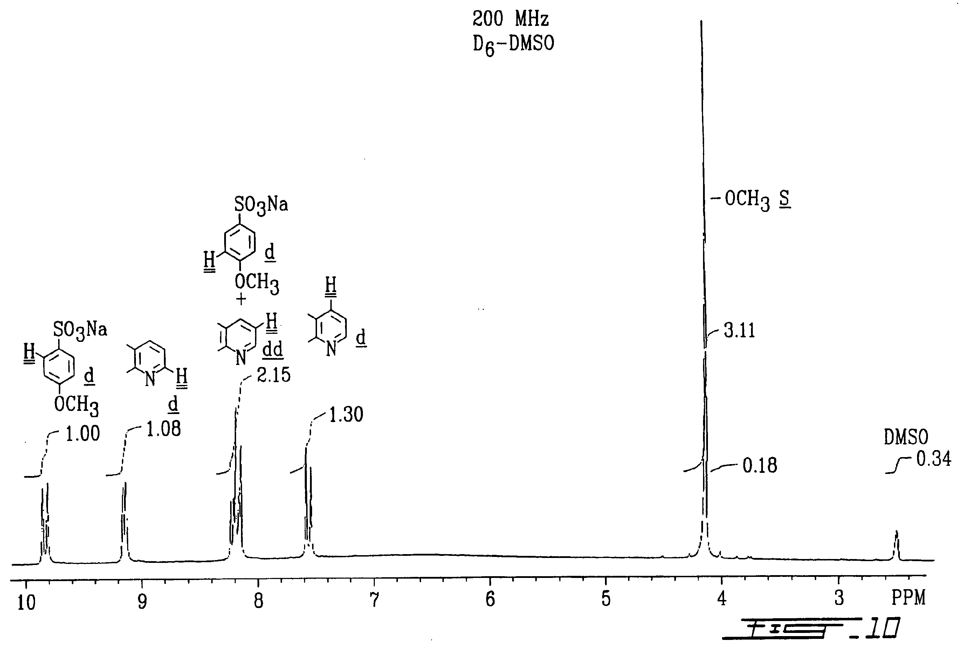

8-Methoxy-5-quinolinesulfonyl chloride (773 mg, see above) was treated with a solution of sodium hydroxide (120 mg) in water at 50°C for 12h. The resulting sodium salt (700 mg) was recrystallized from H₂O to give the title compound as a yellow powder (300 mg). The NMR spectrum of 8-Methoxy-5-quinolinesulfonyl chloride, sodium salt (in deuterated DMSO) is shown in Figure 10.

## Claims

1. Therapeutic compound or pharmaceutically acceptable salt thereof for use in the treatment or prevention of amyloidosis,
wherein the therapeutic compound has the formula: 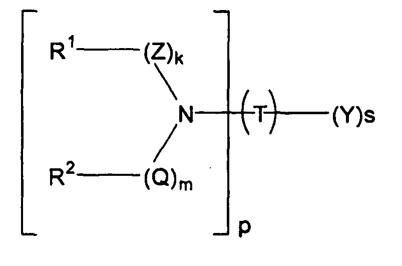 wherein
R¹ is
a straight or branched C₁₋₂₂ alkyl group, a straight or branched C₁₋₂₂ alkenyl group, a straight or branched C₁₋₂₂ alkynyl group, each of which may be substituted on one or more carbons with a halogen, a hydroxyl, a thiol, an amino, an alkoxy, an alkylcarboxy, an alkylthio or a nitro group, or
an alicyclic group selected from cyclopropane, cyclopentane, cycloolefins and fused ring structures, each of which alicyclic groups may be substituted with one or more substituents selected from C₁₋₆ alkyl, C₁₋₆ alkoxy, C₁₋₆ alkylthio, C₁₋₆ alkylamino, C₁₋₆ alkylcarboxyl, nitro, hydroxyl, -CF₃, and -CN, or
a heterocyclic group, which is saturated or unsaturated, can include fused ring structures and
can be substituted at one or more constituent atoms with a halogen, a C₁₋₆ alkyl, a C₁₋₆ alkenyl, a C₁₋₆ alkoxy, a C₁₋₆ alkylthio, a C₁₋₆ alkylamino, a C₁₋₆ alkylcarboxyl, a nitro, a hydroxyl, -CF₃, or -CN, or
an aryl group selected from 5-membered single-ring groups which may include from zero to four heteroatoms and 6-membered single-ring groups selected from benzene, pyrazine, pyridazine and pyrimidine, wherein said aryl group may be substituted at one or more ring positions with substituents selected from halogen, C₁₋₆ alkyl, C₁₋₆ alkenyl, C₁₋₆ alkoxy, C₁₋₆
alkylthio, C₁₋₆ alkylamino, C₁₋₆ alkylcarboxyl, nitro, hydroxyl, -CF₃, and -CN;
R² is a hydrogen atom or a substituted or unsubstituted aliphatic or aryl group;
Z and Q are each independently a carbonyl (C=O), thiocarbonyl (C=S), sulfonyl (SO₂), or sulfoxide (S=O);
k and m are 0 or 1, provided when m is 1, R² is not a hydrogen atom;
p and s are each independently 1 to 2;
T is a group of the formula -(CD¹D²)ₙ-, wherein n is an integer from 1 to 25, C is carbon and D¹ and D² are independently hydrogen or halogen atoms; aliphatic, aromatic or heterocyclic groups; alkylamino or arylamino; or alkyloxy or aryloxy;
or R¹ and R² are taken together to form an alkylene group, k and m are zero, p and s are each one, and T is an alkylene group; and
Y is -A-X, wherein A is SO₃ and X is hydrogen or a cation.

2. The compound according to claim 1, wherein the amyloidosis is primary, secondary, familial or isolated amyloidosis.

3. The compound according to claim 1, wherein R¹ is an alkyl, an alkenyl, or an aryl group each of which as defined in claim 1, k and m are zero, p and s are each one, and R² is a hydrogen or an alkyl group.

4. The compound according to claim 1, wherein the compound is selected from the group consisting of 3-phenylamino-1-propanesulfoaic acid sodium salt sodium salt 3-benzoylaminopropanesulfonic acid, 2-deoxy-2-(3-sulfopropyl)amino-D-glucos, 1-phenyl-2,3,-dimethyl-4-methylamino-pyrazolon-5-*N-*methylsulfonic acid, 3-[(-3,5-dimethyl-1-adamantyl)-amino]-1-propanesulfonic acid, 3-(2-hydroxyethyl)amino-1-propanesulfonic acid, 3-(3-hydroxy-1-propyl)amino-1-propanesulfonic acid, (-)-3-[(*R*)-2-hydroxy-1-propyl]amino-1-propanesulfonic acid, 3-[(d,l)-1-hydroxy-2-propylamino]-1-propanesulfonic acid, 3-(4-hydroxy-1-butyl)amino-1-propanesulfonic acid, 3-(5-hydrox-1-pentyl)amino-1-propanesulfonic acid, 3-(6-hydroxy-1-hexyl)amino-1-propanesulfonic acid, 3-(4-hydroxyphenyl)amino-1-propanesulfonic acid, (÷)-3-[(*S*)-2-hydroxy-1-propyl]amino-1-propanesulfonic acid, (+)-3-[(*S*)-1-hydroxy-2-propyl]amino-1-propanesulfonic acid, (-)-3-[(*R*)-1-hydroxy-2-propyl]amino-1-propanesulfonic acid, (+)-3-[(*S*)-1-hydroxy-2-butyl]amino-1-propanesulfonic acid, (-)-3-[(*R*)-1-hydroxy-2-butyl]amino-1-propanesulfonic acid, 3-[(*dl*)-5-hydroxy-2-pentyl]amino-1-propanesulfonic acid, 3-[(*dl*)-6-hydroxy-2-hexyl]amino-1-propanesulfonic acid, 3-(1-hydroxymethyl-1-cyclopentyl)amino-1- propanesulfonic acid, 3-dimethylamino-1- propanesulfonic acid, 3-amylamino-1-propanesulfonic acid, 3-hexylamino-1-propanesulfonic acid, 3-heptylamino-1-propanesulfonic acid, 3-octylamino-1-propanesulfonic acid, 3-nonylamino-1-propanesulfonic acid, 3-decylamino-1-propanesulfonic acid, 3-undecylamino-1-propanesulfonic acid, 3-dodecylamino-1-propanesulfonic acid, 3-tridecylamino-1-propanesulfonic acid, 3-tetradecylamino-1-propanesulfonic acid, 3-hexadecylamino-1-propanesulfonic acid, 3-octadecylamino-1-propanesulfonic acid, and pharmaceutically acceptable salts thereof.

5. The compound according to claim 1, wherein R¹ is an alkyl, an alkenyl, or an aryl group, each of which is as specified in claim 1, R² is a hydrogen atom or an alkyl group, k is 1, Z is a carbonyl group p and s are each one, and m is zero.

6. The compound according to claim 5, wherein the compound is a mono-*N*-acylated compound.

7. The compound according to claim 5, wherein the compound is selected from the group consisting of 3-acetylamino-1-propanesulfonic acid, 2-acrylamido-2-methyl-1-propanesulfonic acid, 3-benzoylamino-1-propanesulfonic acid, and pharmaceutically acceptable salts thereof.

8. The compound according to claim 1, wherein R¹ is a fused ring structure.

9. The compound according to claim 8, wherein the compound is selected from the group consisting of 3-[(-3,5-dimethyl-1-adamantyl)-amino]-1-propanesulfonic acid and pharmaceutically acceptable salts thereof.

10. The compound according to claim 1, wherein the compound prevents or inhibits Aβ fibril formation.

11. The compound according to claim 10, wherein the compound is selected from the group consisting of 3-[(-3,5-dimethyl-1-adamantyl)-amino]-1-propanesulfonic acid, 2-acrylamido-2-methyl-1-propanesulfonic acid, and pharmaceutically acceptable salts thereof.

12. The compound according to claim 10, wherein the compound is selected from the group consisting of 3-acetylamino-1-propanesulfonic acid, 3-benzoylamino-1-propanesulfonic acid, 3-[(*dl*)-5-Hydroxy-2-pentyl]amino-1-propanesulfonic acid, and pharmaceutically acceptable salts thereof.

13. The compound according to any of the preceding claims, wherein the amyloidosis is Alzheimer's disease, Down's syndrome, or hereditary cerebral hemorrhage amyloidosis.

14. The therapentic compound or a pharmaceutically acceptable salt thereof according to claim 1 that prevents or inhibits Aβ fibril formation
wherein the therapentic compound is selected from the group consisting of 3-[(-3,5-dimethyl-1-adamantyl)-amino]-1-propanesulfonic acid, 2-acrylamido-2-methyl-1-propanesulfonic acid, 3-[2-(1,2,3,4-tetrahydroisoquinolinyl)]-1-propanesulfonic acid, 3-[1-(1,2,3,4-tetrahydroisoquinolinyl)]-1-propanesulfonic acid, 3-(1-indolinyl)-1-propanesulfonic acid, 3-[2-(6-methoxy-1,2,3,4-tetrahydroisoquinolinyl)]-1-propanesulfonic acid, 3-acetylamino-1-propanesulfonic acid, 3-benzoylamino-1-propanesulfonic acid, (+)-3-[(*S*)-2-hydroxy-1-propyl]amino-1-propanesulfonic acid, and pharmaceutically acceptable salts thereof.

15. The compound according to claim 14, wherein the amyloidosis is Alzheimer's disease, Down's syndrome, or hereditary cerebral hemorrhage amyloidosis.

16. The compound according to any of the preceding claims, wherein the compound modulates, prevents or inhibits amyloid aggregation.

17. The compound according to any of the preceding claims, wherein the compound inhibits further amyloid aggregation in a subject with ongoing amyloidosis.

18. The compound according to any of the preceding claims, wherein the disease state is amyloid aggregation associated with Alzheimer's disease.

19. The compound according to any of the preceding claims, wherein the compound inhibits, reduces or disrupts amyloid deposits.

20. The compound according to any of the preceding claims, wherein the compound modulates amyloid associated damage to cells in the subject.

21. The compound according to any of the preceding claims, wherein the amyloidosis is associated with a protein selected from the group consisting of β-amyloid, amyloid A, amyloid κ L-chain, amyloid λ L-chain, a Aβ2M-amyloid, ATTR, AIAPP or amylin, atrial natriuretic factor, procalcitonin, gelsolin, cystatin C, AApoA-I, AApoA-II , fibrinogen, lysozyme, AScr, and PrP-27.

22. The compound according to any of claims 1-12, 14, 16, 17, and 19-21, wherein the amyloidosis is selected from the group consisting of Alzheimer's Disease, Down's syndrome, cerebral hemorrhage, reactive [secondary] amyloidosis, familial Mediterranean Fever, familial amyloid nephrophathy with urticaria and deafness [Muckle-Wells syndrome], idiopathic [primary], myeloma-associated, macroglobulinemia-associated, familial amyloid polyneuropathy, familial amyloid cardiomyopathy, isolated cardiac amyloidosis, systemic senile amyloidosis, adult onset diabetes, insulinoma, isolated atrial amyloidosis, medullary carcinoma of the thyroid, familial amyloidosis, cerebral hemorrhage with amyloidosis , familial amyloidotic polyneuropathy, accelerated senescence in mice, fibrinogen-associated amyloidosis, lysozyme-associated amyloidosis, scrapie, Creutzfeldt-Jacob disease, Gerstmann-Straussler-Scheinker syndrome, and bovine spongiform encephalitis, and transmissible spongiform encephalitis(TSE).

23. The compound according to any of the preceding claims, wherein the amyloidosis is associated with a protein selected from the group consisting of β-amyloid, amyloid A, and IAPP.

24. The compound according to any one of the preceding claims, wherein the compound is to be used in the form of a medicament adapted for oral or intravenous administration.

25. The compound according to any one of the preceding claims, wherein the compound is to be used in the form of a medicament further comprising a pharmaceutically acceptable vehicle.

26. The compound according to claim 1, wherein R¹ is a C₁₋₆ alkyl group, R² a C₁₋₆ alkyl group and T a C₁₋₆ alkylene group.

27. The compound according to claim 26 wherein R¹ is a methyl, ethyl, or propyl group, R² is a methyl, ethyl or propyl group and T is an ethylene, propylene or butylene group.

28. The compound according to any one of claims 26-27, wherein the compound is selected from the group consisting of 3-dimethylamino-1-propanesulfonic acid and pharmaceutically acceptable salts thereof.

29. The compound according to claim 28, wherein the compound is 3-dimethylamino-1-propanesulfonic acid.

30. The compound according to any one of claims 26-29, wherein the compound prevents or inhibits IAPP-associated fibril formation.

31. The compound according to any one of claims 26-39, wherein the amyloidosis is adult onset diabetes.

32. Compound or pharmaceutically acceptable salt thereof, wherein the compound is selected from 4-Phenyl-1-(3'-sulfopropyl)-1,2,3,6-tetrahydropyridine and 2-(3-Sulfobutyl)-1,2,3,4-tetrahydro-9H-pyrido[3,4-b]indole.

## Patentansprüche

1. Therapeutische Verbindung oder pharmazeutisch akzeptables Salz hiervon zur Verwendung bei der Behandlung oder Vorbeugung von Amyloidose,
worin die therapeutische Verbindung die Formel aufweist: 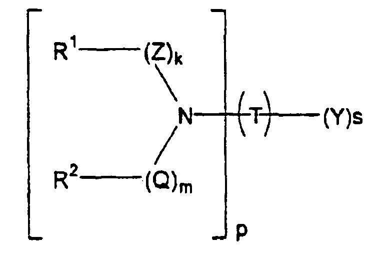 worin
R¹
eine geradkettige oder verzweigte C₁₋₂₂-Alkylgruppe, eine geradkettige oder verzweigte C₁₋₂₂-Alkenylgruppe, eine geradkettige oder verzweigte C₁₋₂₂-Alkinylgruppe, von denen jede an einem oder mehreren Kohlenstoff(en) mit einer Halogen-, einer Hydroxyl-, einer Thiol-, einer Amino-, einer Alkoxy-, einer Alkylcarboxy-, einer Alkylthio- oder einer Nitrogruppe substituiert sein kann, oder
eine alicyclische Gruppe, ausgewählt aus Cyclopropan, Cyclopentan, Cycloolefinen und kondensierten Ringstrukturen, wobei jede von den alicyclischen Gruppen mit einem oder mehreren Substituenten, ausgewählt aus C₁₋₆-Alkyl, C₁₋₆-Alkoxy, C₁₋₆-Alkylthio, C₁₋₆-Alkylamino, C₁₋₆-Alkylcarboxyl, Nitro, Hydroxyl, -CF₃ oder -CN, substituiert sein kann, oder
eine heterocyclische Gruppe, die gesättigt oder ungesättigt ist, kondensierte Ringstrukturen einschließen kann und an einem oder mehreren Aufbauatomen mit einer Halogen-, einer C₁₋₆-Alkyl-, einer C₁₋₆-Alkenyl-, einer C₁₋₆-Alkoxy-, einer C₁₋₆-Alkylthio-, einer C₁₋₆-Alkylamino-, einer C₁₋₆-Alkylcarboxyl-, einer Nitro-, einer Hydroxylgruppe, -CF₃ und -CN substituiert sein kann, oder
eine Arylgruppe, ausgewählt aus 5-gliedrigen Einfach-Ringgruppen, die von 0 bis 4 Heteroatomen umfassen können, und 6-gliedrigen Einfach-Ringgruppen, ausgewählt aus Benzol, Pyrazin, Pyridazin und Pyrimidin, worin die Arylgruppe an einer oder mehr Ringpositionen mit Substituenten, ausgewählt aus Halogen, C₁₋₆-Alkyl, C₁₋₆-Alkenyl, C₁₋₆-Alkoxy, C₁₋₆-Alkylthio, C₁₋₆-Alkylamino, C₁₋₆-Alkylcarboxyl, Nitro, Hydroxy, -CH₃ und -CN substituiert sein kann;
ist,
R² ein Wasserstoffatom oder eine substituierte oder unsubstituierte aliphatische oder Arylgruppe ist;
Z und Q jeweils unabhängig voneinander Carbonyl (C=O), Thiocarbonyl (C=S), Sulfonyl (SO₂) oder Sulfoxid (S=O) darstellen;
k und m 0 oder 1 sind, vorausgesetzt, dass R² kein Wasserstoffatom ist, wenn m 1 ist;
p und s jeweils unabhängig voneinander 1 bis 2 darstellen; T eine Gruppe der Formel -(CD¹D²)ₙ- ist, worin n eine ganze Zahl von 1 bis 25 ist, C Kohlenstoff ist und D¹ und D² jeweils unabhängig voneinander Wasserstoff- oder Halogenatome; aliphatische, aromatische oder heterocyclische Gruppen; Alkylamino oder Arylamino; oder Alkyloxy oder Aryloxy darstellen;
oder R¹ und R² zusammen eine Alkylengruppe bilden, k und m 0 sind, p und s jeweils 1 sind und T eine Alkylengruppe ist; und
Y -A-X ist, worin A SO₃ ist und X Wasserstoff oder ein Kation ist.

2. Verbindung gemäß Anspruch 1, worin die Amyloidose primäre, sekundäre, familiäre oder isolierte Amyloidose ist.

3. Verbindung gemäß Anspruch 1, worin R¹ eine Alkyl-, eine Alkenyl- oder eine Arylgruppe ist, von denen jede wie in Anspruch 1 definiert ist, k und m 0 sind, p und s jeweils 1 sind und R² Wasserstoff oder eine Alkylgruppe ist.

4. Verbindung gemäß Anspruch 1, worin die Verbindung ausgewählt ist aus der Gruppe, bestehend aus 3-Phenylamino-1-propansulfonsäurenatriumsalz, 3-Benzoylaminopropansulfonsäure, 2-Deoxy-2-(3-sulfopropyl)amino-D-glucose, 1-Phenyl-2,3-dimethyl-4-methylamino-pyrazolon-5-N-methylsulfonsäure, 3-[(3,5-Dimethyl-1-adamantyl)amino]-1-propansulfonsäure, 3-(2-Hydroxyethyl)amino-1-propansulfonsäure, 3-(3-Hydroxy-1-propyl)amino-1-propansulfonsäure, (-)-3-[(R)-2-Hydroxy-1-propylamino]-1-propansulfonsäure, 3-[d,1)-1-Hydroxy-2-propylamino]-1-propansulfonsäure, 3-(4-Hydroxy-1-butyl)amino-1-propansulfonsäure, 3-(5-Hydroxy-1-pentyl)amino-1-propansulfonsäure, 3-(6-Hydroxy-1-hexyl)amino-1-propansulfonsäure, 3-(4-Hydroxyphenyl)amino-1-propansulfonsäure, (+)-3-[(S)-2-Hydroxy-1-propyl]amino-1-propansulfonsäure, (+)-3-[(S)-1-Hydroxy-2-propyl]amino-1-propansulfonsäure, (-)-3-[(R)-1-Hydroxy-2-propyl]amino-1-propansulfonsäure, (+)-3-[(S)-1-Hydroxy-2-butyl]amino-1-propansulfonsäure, (-)-3-[(R)-1-Hydroxy-2-butyl]amino-1-propansulfonsäure, 3-[(dl)-5-Hydroxy-2-pentyl]amino-1-propansulfonsäure, 3-[(dl)-6-Hydroxy-2-hexyl]amino-1-propansulfonsäure, 3-(1-Hydroxymethyl-1-cyclpentyl)amino-1-propansulfonsäure, 3-Dimethylamino-1-propansulfonsäure, 3-Amylamino-1-propansülfonsäure, 3-Hexylamino-1-propansulfonsäure, 3-Heptylamino-1-propansulfonsäure, 3-Octylamino-1-propansulfonsäure, 3-Nonylamino-1-propansulfonsäure, 3-Decylamino-1-propansulfonsäure, 3-Undecylamino-1-propansulfonsäure, 3-Dodecylamino-1-propansulfonsäure, 3-Tridecylamino-1-propansulfonsäure, 3-Tetradecylamino-1-propansulfonsäure, 3-Hexadecylamino-1-propansulfonsäure, 3-Octadecylamino-1-propansulfonsäure und pharmazeutisch akzeptablen Salze hiervon.

5. Verbindung gemäß Anspruch 1, worin R¹ eine Alkyl-, eine Alkenyl- oder eine Arylgruppe ist, von denen jede wie in Anspruch 1 definiert ist, R² ein Wasserstoffatom oder eine Alkylgruppe ist, k 1 ist, Z eine Carbonylgruppe ist, p und s jeweils 1 sind, und m 0 ist.

6. Verbindung gemäß Anspruch 5, worin die Verbindung eine mono-N-acylierte Verbindung ist.

7. Verbindung gemäß Anspruch 5, worin die Verbindung ausgewählt ist aus der Gruppe, bestehend aus 3-Acetylamino-1-propansulfonsäure, 2-Acrylamido-2-methyl-1-propansulfonsäure, 3-Benzoylamino-1-propansulfonsäure und pharmazeutisch akzeptablen Salzen hiervon.

8. Verbindung gemäß Anspruch 1, worin R¹ eine kondensierte Ringstruktur ist.

9. Verbindung gemäß Anspruch 8, worin die Verbindung ausgewählt ist aus der Gruppe, bestehend aus 3-[(3,5-Dimethyl-1-adamantyl)amino]-1-propansulfonsäure und pharmazeutisch akzeptablen Salzen hiervon.

10. Verbindung gemäß Anspruch 1, worin die Verbindung die Aß-Fibrillenbildung vermeidet oder inhibiert.

11. Verbindung gemäß Anspruch 10, worin die Verbindung ausgewählt ist aus der Gruppe, bestehend aus 3-[(3,5-Dimethyl-1-adamantyl)amino]-1-propansulfonsäure, 2-Acrylamido-2-methyl-1-propansulfonsäure und pharmazeutisch akzeptablen Salzen hiervon.

12. Verbindung gemäß Anspruch 10, worin die Verbindung ausgewählt ist der Gruppe, bestehend aus 3-Acetylamino-1-propansulfonsäure, 3-Benzoylamino-1-propansulfonsäure, 3-[(dl)-5-Hydroxy-4-pentyl]amino-1-propansulfonsäue und pharmazeutisch akzeptablen Salzen hiervon.

13. Verbindung gemäß irgendeinem vorhergehenden Anspruch,
worin die Amyloidose die Alzheimer-Krankheit, das Down-Syndrom oder vererbbare Gehirnblutungs-Amyloidose ist.

14. Therapeutische Verbindung oder pharmazeutisch akzeptables Salz hiervon, das die Bildung von Aβ-Fibrillen vermeidet oder inhibiert, worin die therapeutische Verbindung ausgewählt ist aus der Gruppe, bestehend aus 3-[(3,5-Dimethyl-1-adamantyl)amino]-1-propansulfonsäure, 2-Acrylamido-2-methyl-1-propansulfonsäure, 3-[2-(1,2,3,4-Tetrahydroisochinolinyl)]-1-propansulfonsäure, 3-[1-(1,2,3,4-Tetrahydroisochinolinyl)]-1-propansulfonsäure, 3-(1-Indolinyl)-1-propansulfonsäure, 3-[2-(6-Methoxy-1,2,3,4-tetrahydroisochinolinyl)]-1-propansulfonsäure, 3-Acetylamino-1-propansulfonsäure, 3-Benzoylamino-1-propansulfonsäure, (+)-3-[(S)-2-Hydroxy-1-propyl]amino-1-propansulfonsure und pharmazeutisch akzeptablen Salzen hiervon.

15. Verbindung gemäß Anspruch 14, worin die Amyloidose die Alzheimer-Krankheit, das Down-Syndrom oder vererbbare Gehirnblutungs-Amyloidose ist.

16. Verbindung gemäß irgendeinem der vorhergehenden Ansprüche, worin die Verbindung die Amyloidaggregation moduliert, vermeidet oder inhibiert.

17. Verbindung gemäß irgendeinem der vorhergehenden Ansprüche, worin die Verbindung eine weitere Amyloidaggregation in einem Patienten mit bestehender Amyloidose inhibiert.

18. Verbindung gemäß irgendeinem der vorhergehenden Ansprüche, worin der Krankheitszustand eine Amyloidaggregation ist, die mit der Alzheimer-Krankheit assoziiert ist.

19. Verbindung gemäß irgendeinem der vorhergehenden Ansprüche, worin die Verbindung Amyloidablagerungen inhibiert, verringert oder zerstört.

20. Verbindung gemäß irgendeinem der vorhergehenden Ansprüche, worin die Verbindung das Amyloid moduliert, das mit einer Zellschädigung in dem Patienten in Zusammenhang steht.

21. Verbindung gemäß irgendeinem der vorhergehenden Ansprüche, worin die Amyloidose mit einem Protein in Zusammenhang steht, das ausgewählt ist aus der Gruppe, bestehend aus β-Amyloid, Amyloid A, Amyloid κ L-Kette, Amyloid λ L-Kette, ein Aß2M-Amyloid, ATTR, AIAPP oder Amylin, atrialer natriuretischer Faktor, Procalcitonin, Gelsolin, Cystatin C, AApoA-I, AApoA-II, Fibrinogen, Lysozym, AScr und PrP-27.

22. Verbindung gemäß irgendeinem der Ansprüche 1 bis 12, 14, 16, 17 und 19 bis 21, worin die Amyloidose ausgewählt ist aus der Gruppe, bestehend aus der Alzheimer-Krankheit, dem Down-Syndrom, Hirnblutung, reaktiver [sekundärer] Amyloidose, familiärem Mittelmeerfiber, familiärer Amyloid-Nephrophathie mit Nesselfiber und Taubheit [Muckle-Wells-Syndrom], idiopathischer [primärer], Myelom-assoziierter, Makroglobulinämieassoziierter, familiärer Amyloid-Polyneuropathie, familiärer Amyloid-Kardiomyopathie, isolierter kardialer Amyloidose, systemischer seniler Amyloidose, Erwachsenendiabetes, Insulinoma, isolierter atrialer Amyloidose, medulärem Schilddrüsenkrebs, familiärer Amyloidose, Hirnblutung mit Amyloidose, familiärer amyloidotischer Polyneuropathie, beschleunigter Vergreisung in Mäusen, mit Fibrinogen assoziierte Amyloidose, mit Lysozym assoziierte Amyloidose, Traberkrankheit, Creutzfeldt-Jakob-Krankheit, Gerstmann-Straussler-Scheinker-Syndrom und Rinderwahn, und übertragbarer spongiöser Encephalitis (TSE).

23. Verbindung gemäß irgendeinem der vorhergehenden Ansprüche, worin die Amyloidose mit einem Protein assoziiert ist, das ausgewählt ist aus der Gruppe, bestehend aus β-Amyloid, Amyloid A und IAPP.

24. Verbindung gemäß irgendeinem der vorhergehenden Ansprüche, worin die Verbindung in der Form eines Medikaments einzusetzen ist, das für die orale oder intravenöse Verabreichung eingerichtet ist.

25. Verbindung gemäß irgendeinem der vorhergehenden Ansprüche, worin die Verbindung in der Form eines Medikaments zu verwenden ist, das ferner ein pharmazeutisch akzeptables Vehikel umfasst.

26. Verbindung gemäß Anspruch 1, worin R¹ eine C₁₋₆-Alkylgruppe ist, R² eine C₁₋₆Alkylgruppe ist und T eine C₁₋₆-Alkylengruppe ist.

27. Verbindung gemäß Anspruch 26, worin R¹ eine Methyl-, Etyhl- oder Propylgruppe ist, R² eine Methyl-, Ethyl- oder Propylgruppe ist und T eine Ethylen-, Propylen- oder Butylengruppe ist.

28. Verbindung gemäß irgendeinem der Ansprüche 26 bis 27,
worin die Verbindung ausgewählt ist aus der Gruppe, bestehend aus 3-Dimethylamino-1-propansulfonsäure und pharmazeutisch akzeptablen Salzen hiervon.

29. Verbindung gemäß Anspruch 28, worin die Verbindung 3-Dimethylamino-1-propansulfonsäure ist.

30. Verbindung gemäß irgendeinem der Ansprüche 26 bis 29,
worin die Verbindung die mit IAPP assoziierte Fibrillenbildung vermeidet oder inhibiert.

31. Verbindung gemäß irgendeinem der Ansprüche 26 bis 30,
worin die Amyloidose Erwachsenendiabetes ist.

32. Verbindung oder pharmazeutisch akzeptables Salz hiervon, worin die Verbindung ausgewählt ist aus 4-Phenyl-1-(3'-sulfopropyl)-1,2,3,6-tetrahydropyridin und 2-(3-Sulfobutyl)-1,2,3,4-tetrahydro-9H-pyrido[3,4-b]indol.

## Revendications

1. Composé thérapeutique ou sel pharmaceutiquement acceptable de celui-ci pour une utilisation dans le traitement ou la prévention de l'amyloïdose,
dans lequel le composé thérapeutique possède la formule 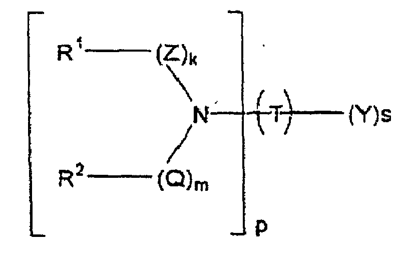 dans laquelle
R¹ est
un groupe alkyle en C₁₋₂₂ linéaire ou ramifié, un groupe alcényle en C₁₋₂₂ linéaire ou ramifié, un groupe alcynyle en C₁₋₂₂ linéaire ou ramifié, qui peuvent chacun être substitués sur un ou plusieurs carbone(s) par un atome d'halogène, un groupe hydroxyle, un groupe thiol, un groupe amino, un groupe alcoxy, un groupe alkylcarboxy, un groupe alkylthio, ou un groupe nitro, ou
un groupe alicyclique choisi parmi le cyclopropane, le cyclopentane, les cyclooléfines et les structures à cycle condensé, chacun des groupes alicycliques peut être substitué par un ou plusieurs substituant(s) choisi(s) parmi un groupe alkyle en C₁₋₆, alcoxy en C₁₋₆, alkylthio en C₁₋₆, alkylamino en C₁₋₆, alkylcarboxyle en C₁₋₆, nitro, hydroxyle, -CF₃, et -CN, ou
un groupe hétérocyclique, lequel est saturé ou insaturé, peut inclure des structures à cycle condensé et peut être substitué au niveau d'un ou plusieurs atome(s) constitutif(s) par un atome d'halogène, un alkyle en C₁₋₆, un alcényle en C₁₋₆, un alcoxy en C₁₋₆, un alkylthio en C₁₋₆, un alkylamino en C₁₋₆, un alkylcarboxyle en C₁₋₆, un nitro, un hydroxyle, -CF₃, et -CN, ou
un groupe aryle choisi parmi les groupes à cycle unique à 5 membres qui peut inclure de zéro à quatre hétéroatome(s) et les groupes à cycle unique à 6 membres choisis parmi le benzène, la pyrazine, la pyridazine et la pyrimidine, dans lequel ledit groupe aryle peut être substitué au niveau d'une ou plusieurs position(s) du cycle par des substituants choisis parmi un atome d'halogène, un groupe alkyle en C₁₋₆, alcényle en C₁₋₆, alcoxy en C₁₋₆, alkylthio en C₁₋₆, alkylamino en C₁₋₆, alkylcarboxyle en C₁₋₆, nitro, hydroxyle, -CF₃, et -CN ;
R² est un atome d'hydrogène ou un groupe aliphatique ou aryle substitué ou non substitué ;
Z et Q sont chacun, de manière indépendante, un carbonyle (C=O), thiocarbonyle (C=S), sulfonyle (SO₂), ou sulfoxyde (S=O) ;
k et m valent 0 ou 1, à condition que lorsque m vaut 1, R² ne soit pas un atome d'hydrogène ;
p et s valent chacun, de manière indépendante, 1 à 2 ;
T est un groupe de la formule (CD¹D²)ₙ₋, dans laquelle n est un nombre entier de 1 à 25, C est un carbone et D¹ et D² sont, de manière indépendante, des atomes d'hydrogène ou d'halogène ; des groupes aliphatiques, aromatiques ou hétérocycliques ; un groupe alkylamino ou arylamino ; ou alkyloxy ou aryloxy ;
ou bien R¹ et R² sont pris ensemble pour former un groupe alkylène, k et m valent 0, p et s valent chacun un, et T est un groupe alkylène ; et
Y est -A-X, dans lequel A est SO₃ et X est un atome d'hydrogène ou un cation.

2. Composé selon la revendication 1, dans lequel l'amyloïdose est une amyloïdose primaire, secondaire, familiale ou isolée.

3. Composé selon la revendication 1, dans lequel R¹ est un groupe alkyle, un groupe alcényle, ou un groupe aryle, qui sont chacun tel que défini selon la revendication 1, k et m valent zéro, p et s valent chacun un, et R² est un atome d'hydrogène ou un groupe alkyle.

4. Composé selon la revendication 1, dans lequel le composé est choisi dans le groupe constitué du sel sodique d'acide 3-phénylamino-1-propanesulfonique, de l'acide 3-benzoylaminopropanesulfonique, du 2-désoxy-2-(3-sulfopropyl)amino-D-glucose, de l'acide 1-phényl-2,3-diméthyl-4-méthylamino-pyrazolon-5-N-méthylsulfonique, l'acide 3-[(3,5-diméthyl-1-adamantyl)-amino]-1-propanesulfonique, l'acide 3-(2-hydroxyéthyl)amino-1-propanesulfonique, l'acide 3-(3-hydroxy-1-propyl)amino-1-propanesulfonique, l'acide (-)-3-[(R)-2-hydroxy-1-propyl]amino-1-propanesulfonique, l'acide 3-[(d,l)-1-hydroxy-2-propyl]amino-1-propanesulfonique, l'acide 3-(4-hydroxy-1-butyl)amino-1-propanesulfonique, l'acide 3-(5-hydroxy-1-pentyl)amino-1-propanesulfonique, l'acide 3-(6-hydroxy-1-hexyl)amino-1-propanesulfonique, l'acide 3-(4-hydroxyphényl)amino-1-propanesulfonique, l'acide (+)-3-[(*S*)-2-hydroxy-1-propyl]amino-1-propanesulfonique, l'acide (+)-3-[(*S*)-1-hydroxy-2-propyl]amino-1-propanesulfonique, l'acide (-)-3-[(*R*)-1-hydroxy-2-propyl]amino-1-propanesulfonique, l'acide (+)-3-[(*S*)-1-hydroxy-2-butyl]amino-1-propanesulfonique, l'acide (-)-3-[(*R*)-1-hydroxy-2-butyl]amino-1-propanesulfonique, l'acide 3-[(*dl*)-5-hydroxy-2-pentyl]amino-1-propanesulfonique, l'acide 3-[(*dl*)-6-hydroxy-2-hexyl]amino-1-propanesulfonique, l'acide 3-(1-hydroxyméthyl-1-cyclopentyl)amino-1-propanesulfonique, l'acide 3-diméthylamino-1-propanesulfonique, l'acide 3-amylamino-1-propanesulfonique, l'acide 3-hexylamino-1-propanesulfonique, l'acide 3-heptylamino-1-propanesulfonique, l'acide 3-octylamino-1-propanesulfonique, l'acide 3-nonylamino-1-propanesulfonique, l'acide 3-décylamino-1-propanesulfonique, l'acide 3-undécylamino-1-propanesulfonique, l'acide 3-dodécylamino-1-propanesulfonique, l'acide 3-tridécylamino-1-propanesulfonique, l'acide 3-tétradécylamino-1-propanesulfonique, l'acide 3-hexadécylamino-1-propanesulfonique, l'acide 3-octadécylamino-1-propanesulfonique, et des sels pharmaceutiquement acceptables de ceux-ci.

5. Composé selon la revendication 1, dans lequel R¹ est un groupe alkyle, un groupe alcényle, ou un groupe aryle, qui sont chacun tel que spécifié selon la revendication 1, R² est un atome d'hydrogène ou un groupe alkyle, k vaut 1, Z est un groupe carbonyle, p et s valent chacun un, et m vaut zéro.

6. Composé selon la revendication 5, dans lequel le composé est un composé mono-N-acylé.

7. Composé selon la revendication 5, dans lequel le composé est choisi dans le groupe constitué de l'acide 3-acétylamino-1-propanesulfonique, l'acide 2-acrylamido-2-méthyl-1-propanesulfonique, l'acide 3-benzoylamino-1-propanesulfonique, et des sels pharmaceutiquement acceptables de ceux-ci.

8. Composé selon la revendication 1, dans lequel R¹ est une structure à cycle condensé.

9. Composé selon la revendication 8, dans lequel le composé est choisi dans le groupe constitué de l'acide 3-[(-3,5-diméthyl-1-adamantyl)-amino]-1-propanesulfonique et des sels pharmaceutiquement acceptables de celui-ci.

10. Composé selon la revendication 1, dans lequel le composé empêche ou inhibe la formation de fibrilles Aβ.

11. Composé selon la revendication 10, dans lequel le composé est choisi dans le groupe constitué de l'acide 3-[(-3,5-diméthyl-1-adamantyl)-amino]-1-propanesulfonique, l'acide 2-acrylamido-2-méthyl-1-propanesulfonique, et des sels pharmaceutiquement acceptables de ceux-ci.

12. Composé selon la revendication 10, dans lequel le composé est choisi dans le groupe constitué de l'acide 3-acétylamino-1-propanesulfonique, l'acide 3-benzoylamino-1-propanesulfonique, l'acide 3-[(*dl*)-5-hydroxy-2-pentyl]amino-1-propanesulfonique, et des sels pharmaceutiquement acceptables de ceux-ci.

13. Composé selon l'une quelconque des revendications précédentes, dans lequel l'amyloïdose est la maladie d'Alzheimer, le syndrome de Down, ou une amyloïdose hémorragique cérébrale héréditaire.

14. Composé thérapeutique ou sel pharmaceutiquement acceptable de celui-ci selon la revendication 1 qui empêche
ou inhibe la formation de fibrilles Aβ, dans lequel le composé thérapeutique est choisi dans le groupe constitué de l'acide 3-[(3,5-diméthyl-1-adamantyl)-amino]-1-propanesulfonique, l'acide 2-acrylamido-2-méthyl-1-propanesulfonique, l'acide 3-[2-(1,2,3,4-tétrahydroisoquinolinyl)]-1-propanesulfonique, l'acide 3-[1-(1,2,3,4-tétrahydroisoquinolinyl)]-1-propanesulfonique, l'acide 3-(1-indolinyl)-1-propanesulfonique, l'acide 3-[2-(6-méthoxy-1,2,3,4-tétrahydroisoquinolinyl)]-1-propanesulfonique, l'acide 3-acétylamino-1-propanesulfonique, l'acide 3-benzoylamino-1-propanesulfonique, l'acide (+)-3-[(*S*)-2-hydroxy-1-propyl]amino-1-propanesulfonique, et les sels pharmaceutiquement acceptables de ceux-ci.

15. Composé selon la revendication 14, dans lequel l'amyloïdose est la maladie d'Alzheimer, le syndrome de Down, ou une amyloïdose hémorragique cérébrale héréditaire.

16. Composé selon l'une quelconque des revendications précédentes, dans lequel le composé module, empêche ou inhibe l'agrégation d'amyloïde.

17. Composé selon l'une quelconque des revendications précédentes, dans lequel le composé inhibe l'agrégation supplémentaire d'amyloïde chez un sujet atteint d'une amyloïdose progressive.

18. Composé selon l'une quelconque des revendications précédentes, dans lequel l'état pathologique est une agrégation d'amyloïde associée à la maladie d'Alzheimer.

19. Composé selon l'une quelconque des revendications précédentes, dans lequel le composé inhibe, réduit ou détruit les dépôts d'amyloïde.

20. Composé selon l'une quelconque des revendications précédentes, dans lequel le composé module une lésion associée à l'amyloïde, à des cellules chez le sujet.

21. Composé selon l'une quelconque des revendications précédentes, dans lequel l'amyloïdose est associée à une protéine choisie dans le groupe constitué de l'amyloïde β, l'amyloïde A, la chaîne L d'amyloïde *K*, la chaîne L d'amyloïde λ, un amyloïde Aβ2M, l'ATTR, l'ALAPP ou l'amyline, le facteur natriurétique auriculaire, la procalcitonine, la gelsoline, la cystatine C, l'AApoA-I, l'AApoA-II, le fibrinogène, le lysozyme, l'AScr, et la PrP-27.

22. Composé selon l'une quelconque des revendications 1 à 12, 14, 16, 17, et 19 à 21, dans lequel l'amyloïdose est choisie dans le groupe constitué de la maladie d'Alzheimer, le syndrome de Down, une hémorragie cérébrale héréditaire, l'amyloïdose [secondaire] réactive, la fièvre méditerranéenne familiale, la néphropathie amyloïde familiale avec urticaire et surdité [syndrome de Muckle-Wells], l'amyloïdose idiopathique [primaire], associée à un myélome, associée à une macroglobulinémie, la polyneuropathie amyloïde familiale, la cardiomyopathie amyloïde familiale, l'amyloïdose cardiaque isolée, l'amyloïdose sénile systémique, le diabète de la maturité, l'insulinome, l'amyloïdose auriculaire isolée, le carcinome médullaire de la thyroïde, l'amyloïdose familiale, l'hémorragie cérébrale avec amyloïdose, la polyneuropathie amyloïdique familiale, la sénescence accélérée chez la souris, l'amyloïdose associée au fibrinogène, l'amyloïdose associée au lysozyme, la tremblante du mouton, la maladie de Creutzfeldt-Jacob, le syndrome de Gerstmann-Straussler-Scheinker, et l'encéphalopathie bovine spongiforme, et l'encéphalite spongiforme transmissible (TSE).

23. Composé selon l'une quelconque des revendications précédentes, dans lequel l'amyloïdose est associée à une protéine choisie dans le groupe constitué de l'amyloïde β, l'amyloïde A, et l'IAPP.

24. Composé selon l'une quelconque des revendications précédentes, dans lequel le composé doit être utilisé sous la forme d'un médicament adapté à une administration orale ou intraveineuse.

25. Composé selon l'une quelconque des revendications précédentes, dans lequel le composé doit être utilisé sous la forme d'un médicament comprenant en outre un véhicule pharmaceutiquement acceptable.

26. Composé selon la revendication 1, dans lequel R¹ est un groupe alkyle en C₁₋₆, R² est un groupe alkyle en C1-6 et T un groupe alkylène en C1-6.

27. Composé selon la revendication 26, dans lequel R¹ est un groupe méthyle, éthyle, ou propyle, R² est un groupe méthyle, éthyle ou propyle et T est un groupe éthylène, propylène ou butylène.

28. Composé selon l'une quelconque des revendications 26 à 27, dans lequel le composé est choisi dans le groupe constitué de l'acide 3-diméthylamino-1-propanesulfanique et des sels pharmaceutiquement acceptables de celui-ci.

29. Composé selon la revendication 28, dans lequel le composé est l'acide 3-diméthylamino-1-propanesulfonique.

30. Composé selon l'une quelconque des revendications 26 à 29, dans lequel le composé empêche ou inhibe la formation de fibrilles associées à l'IAPP.

31. Composé selon l'une quelconque des revendications 26 à 30, dans lequel l'amyloïdose est un diabète de la maturité.

32. Composé ou sel pharmaceutiquement acceptable de celui-ci, dans lequel le composé est choisi parmi la 4-phényl-1-(3'-sulfopropyl)-1,2,3,6-tétrahydropyridine et le 2-(3-sulfobutyl)-1,2,3,4-tétrahydro-9H-pyrido[3,4-b]indole.
